# EUROPEAN PATENT APPLICATION

(11) **EP 4 785 892 A1**
(43) Date of publication of application: **05.08.2026**
(21) Application number: 26154899.4
(22) Date of filing: 29.01.2026
(51) Int. Cl.: A61B 34/20, A61B 34/30

(54) **INTERACTIVE TECHNIQUES FOR ACTIVATION OF A SURGICAL SYSTEM**

(30) Priority: 04.02.2025 US 202563753553 P
(71) Applicant: MAKO Surgical Corp., Weston, FL 33331 (US)
(72) Inventor: Gonzalez, Jean, Plantation, 33325 (US); Pourghodrat, Sina, Plantation, 33322 (US)
(74) Representative: Schott, Jakob Valentin

(57) **Abstract**

Surgical systems and methods involve a surgical robot configured to support and move a surgical tool, a tracking system configured to track a position of a surgical staff member, and one or more controllers coupled to the surgical robot and the tracking system. The controller(s) define a first virtual zone and a second virtual zone relative to the surgical robot, detect a positioning of the surgical staff member in the first virtual zone based on tracked position data, and in response, trigger a first control mode for the surgical robot. The controller(s) further detect a positioning of the surgical staff member in the second virtual zone and, in response, trigger a second control mode for the surgical robot that is different from the first control mode.

## Description

### CROSS-REFERENCE TO RELATED APPLICATIONS

The subject application claims priority to and all the benefits of United States Provisional Patent App. No. 63/753,553, filed February 4, 2025, the entire contents which are hereby incorporated by reference.

### BACKGROUND

Autonomous robots have been applied commercially to surgical procedures. These systems make precise bone resections, improving implant fit and placement relative to techniques that rely on manual instruments. For instance, the autonomous robot may perform cutting on a patient's anatomy. The cutting may be performed autonomously with a high-speed burr, although the surgeon can monitor progress and interrupt it if necessary. Conventionally, an operator may be required to provide a manual input to a surgical tool (e.g., the high-speed surgical burr) or to the surgical system (e.g., a pendant including a switch) while the autonomous robot is performing a surgical procedure. However, there remains a need in the art to allow an operator to provide such an input, without manually interacting with the surgical tool. Additionally, there remains a need in the art to provide a safe operating environment for autonomous robots.

### SUMMARY

This Summary introduces a selection of concepts in a simplified form that are further described below in the Detailed Description below. This Summary is not intended to limit the scope of the claimed subject matter nor identify key features or essential features of the claimed subject matter.

According to a first aspect, a surgical system is provided, comprising: a surgical robot that is configured to support and move a surgical tool; a tracking system that is configured to track a position of a surgical staff member; and one or more controllers coupled to the surgical robot and the tracking system and being configured to: define a first virtual zone relative to the surgical robot; define a second virtual zone relative to the surgical robot; detect a positioning of the surgical staff member in the first virtual zone based on the position of the surgical staff member tracked by the tracking system; in response to detection of the positioning of the surgical staff member in the first virtual zone, trigger a first control mode for the surgical robot; detect a positioning of the surgical staff member in the second virtual zone based on the position of the surgical staff member tracked by the tracking system; and in response to detection of the positioning of the surgical staff member in the second virtual zone, trigger a second control mode for the surgical robot that is different from the first control mode.

According to a second aspect, a surgical system is provided, comprising: a surgical robot that is configured to support and move a surgical tool; a tracking system that is configured to track a position of a surgical staff member; and one or more controllers coupled to the surgical robot and the tracking system and being configured to: define a first virtual zone relative to the surgical robot; define a second virtual zone relative to the surgical robot; detect a transitional movement of the surgical staff member between the first virtual zone and the second virtual zone based on the position of the surgical staff member tracked by the tracking system; and in response to detection of the transitional movement, trigger a control mode for the surgical robot.

According to a third aspect, a surgical robot that is configured to support and move a surgical tool; a tracking system that is configured to track a position of a surgical staff member; and one or more controllers coupled to the surgical robot and the tracking system and being configured to: define a virtual zone relative to the surgical robot; detect a positioning of an entire body of the surgical staff member in the virtual zone based on the position of the surgical staff member tracked by the tracking system; in response to detection of the positioning of the entire body of the surgical staff member in the virtual zone, trigger a first control mode for the surgical robot; while the surgical staff member is substantially positioned in the virtual zone, detect a positioning of a limb of the surgical staff member outside of the virtual zone based on the position of the surgical staff member tracked by the tracking system; and in response to detection of the positioning of the limb of the surgical staff member outside of the virtual zone, trigger a second control mode for the surgical robot that is different from the first control mode.

According to a fourth aspect, a surgical system is provided, comprising: a surgical robot that is configured to support and move a surgical tool; a head mounted device (HMD) that is configured to be worn by a surgical staff member, wherein the HMD comprises an HMD tracking system that is configured to track a position of the surgical staff member; and one or more controllers coupled to the surgical robot and the HMD and being configured to: define a virtual zone relative to the surgical robot based on input from the HMD tracking system; detect a positioning of the surgical staff member in the virtual zone based on the position of the surgical staff member tracked by the HMD tracking system; and in response to detection of the positioning of the surgical staff member in the virtual zone, trigger a control mode for the surgical robot.

Any of the above aspects may be combined in-part or in-whole with any of the implementations below.

The term "surgical staff member" includes one or more human persons working directly in the operating room (OR) near the surgical robot. The surgical staff member moves around the OR to facilitate the surgical procedure. The surgical staff member can include any professional or team member of the surgical team, including one or more surgeons, doctors, nurses, surgical technicians, surgical product specialists, and the like. Any of the aspects above can be implemented to track and detect positioning of any number of surgical staff members. Moreover, the patient is not one of the surgical staff members. However, features of the patient (e.g., geometric features (body size, joint location), spatial features (location), movement (range of motion), patient-specific surgical plan, patient procedure type, patient demographics, etc.) can be considered by the system of any of the above aspects, for example, to define virtual zones or conditions.

In one implementation, the first control mode and the second control mode each comprise control of the surgical robot and/or surgical tool according to one or more operating parameters, the one or more operating parameters including: a cutting speed of the surgical tool, a feed rate of the surgical tool, and a tool path of the surgical tool. In one implementation, an operating parameter of the first control mode is the same as an operating parameter of the second control mode, and wherein the operating parameter is controlled differently in the first control mode than in the second control mode. In one implementation, an operating parameter of the first control mode is different than an operating parameter of the second control mode.

In one implementation, the first control mode and the second control mode each comprise control of the surgical robot according to an operating mode, the operating mode including one of: an automated mode, a manual mode, and a halt mode; and the operating mode of the first control mode is different than the operating mode of the second control mode. In one implementation, the control mode for the surgical robot comprises an automated mode.

In one implementation, the one or more controllers are configured to define the first virtual zone and the second virtual zone such that the first virtual zone and the second virtual zone are defined relative to a virtual reference point located relative to the surgical robot; and the first virtual zone and the second virtual are non-overlapping. In one implementation, the first virtual zone surrounds the second virtual zone. The virtual zones may be 2-dimensional, 3-dimensional, or combinations thereof. In one implementation, the first virtual zone includes a circular shape, and wherein the second virtual zone extends annularly about the first virtual zone.

In one implementation, the one or more controllers are configured to: define a third virtual zone relative to the surgical robot; detect a positioning of the surgical staff member in the third virtual zone based on the position of the surgical staff member tracked by the tracking system; and in response to detection of the positioning of the surgical staff member in the third virtual zone, trigger a third control mode for the surgical robot that is different from the first control mode and the second control mode.

In one implementation, the one or more controllers are configured to detect a condition related to one or more of: a surgical plan; a step of a surgical procedure; an elapsing of a predetermined amount of time; a patient; the surgical robot; the surgical tool; and the surgical staff member. In one implementation, the one or more controllers are configured to define a feature of the first virtual zone and/or a feature of the second virtual zone based on detection of the condition. Features can include a geometric feature, a spatial feature, a temporal feature, and/or an interaction feature. In one implementation, the one or more controllers are configured to: detect a change in the condition; and in response to detection of the change of the condition, modify the feature of the first virtual zone and/or the feature of the second virtual zone. In one implementation, the one or more controllers are configured to define the first control mode and/or the second control mode based on the condition. In one implementation, the one or more controllers are configured to: detect a change in the condition; and in response to detection of the change of the condition, modify the first control mode and/or the second control mode.

In one implementation, the tracking system comprises a head-mounted device (HMD), and wherein the HMD comprises the one or more controllers. In one implementation, the HMD comprises a display that is positionable in front of the eyes of a user of the HMD, and wherein the HMD is configured to present on the display a virtual representation of the first virtual zone and the second virtual zone combined with a real-world view of the surgical robot. In one implementation, the tracking system comprises a navigation system comprising a camera unit, and wherein the navigation system comprises the one or more controllers. In one implementation, the tracking system comprises a head-mounted device (HMD) and a navigation system comprising a camera unit, and wherein the one or more controllers are coupled to the HMD and to the navigation system.

In one implementation, prior to detection of the transitional movement, the one or more controllers are configured to control the surgical robot according to a halt mode; wherein, in response to detection of the transitional movement, the one or more controllers are configured to transition from the halt mode to the automated mode. In one implementation, the transitional movement is based on an elapsing of a predetermined amount of time. In one implementation, the control mode is further defined as a first control mode, and wherein, prior to detection of the transitional movement, the one or more controllers are configured to control the surgical robot according to a second control mode.

In one implementation, the one or more controllers are configured to: define a third virtual zone relative to the surgical robot, wherein the transitional movement of the surgical staff member is further defined as being movement between the first virtual zone, the second virtual zone, and the third virtual zone; detect the transitional movement of the surgical staff member between the first virtual zone, the second virtual zone, and the third virtual zone based on the position of the surgical staff member tracked by the tracking system; and in response to detection of the transitional movement, trigger the control mode for the surgical robot.

In one implementation, the transitional movement of the surgical staff member is further defined as being movement from the first virtual zone to the second virtual zone and back the first virtual zone.

In one implementation, the one or more controllers are configured to define the first virtual zone and the second virtual zone such that the first virtual zone and the second virtual zone are defined relative to a virtual reference point located relative to the surgical robot; and the second virtual zone virtual zone is located closer to the virtual reference point than the first virtual zone.

In one implementation, the HMD comprises a display that is positionable in front of the eyes of a user of the HMD, and wherein the HMD is configured to present on the display a virtual representation of the virtual zone with a real-world view of the surgical robot. In one implementation, the HMD is configured to present on the display a virtual notification in response to detection of the position of the surgical staff member in the virtual zone. In one implementation, the HMD comprises an input device that is configured to receive an input from the surgical staff member, and the one or more controllers are configured to modify the virtual zone based on the input. In one implementation, the HMD comprises a display that is positionable in front of the eyes of a user of the HMD, and wherein the HMD is configured to present on the display a virtual representation of at least one of the first virtual zone, the second virtual zone, and the third virtual zone with a real-world view of the surgical robot.

In one implementation, the virtual zone is further defined as a first virtual zone, wherein the control mode is further defined as a first control mode, and wherein the one or more controllers are configured to: define a second virtual zone relative to the surgical robot based on input from the HMD tracking system; define a third virtual zone relative to the surgical robot based on input from the HMD tracking system; in response to detection of the positioning of the surgical staff member in the second virtual zone, trigger a second control mode for the surgical robot; and in response to detection of the positioning of the surgical staff member in the third virtual zone, trigger a third control mode for the surgical robot.

### BRIEF DESCRIPTION OF THE DRAWINGS

Advantages of the present invention will be readily appreciated as the same becomes better understood by reference to the following detailed description when considered in connection with the accompanying drawings wherein:
FIG. 1 is a perspective view of a surgical system, according to one implementation.
FIG. 2 is a schematic view of an example control system that can be used with the surgical system.
FIG. 3 is an illustration of various coordinate systems and transforms that can be established relative to the various components of the surgical system, according to one implementation.
FIGS. 4 and 5 are schematic views of example virtual zones defined by the example control system of FIG. 2.
FIG. 6 is a flowchart of a method of triggering a control mode of the surgical system in response to transitional movement of the surgical staff member between virtual zones.
FIG. 7 is a schematic view illustrating an example transitional movement of the surgical staff member between virtual zones.
FIG. 8 is a flowchart of a method of triggering a control mode of the surgical system in response to detecting a positioning of the surgical staff member in a virtual zone.
FIGS. 9A-9C are schematic views illustrating an example positioning of the surgical staff member in a virtual zone.
FIG. 10 is a flowchart of a method of triggering a control mode of the surgical system in response to detecting a positioning of a limb of the surgical staff member outside of a virtual zone, while the surgical staff member is substantially positioned in the virtual zone, according to one implementation.
FIG. 11 is a schematic view illustrating an example positioning of a limb of the surgical staff member outside of a virtual zone, while the surgical staff member is substantially positioned in the virtual zone.

### DETAILED DESCRIPTION

### I. Example System Overview

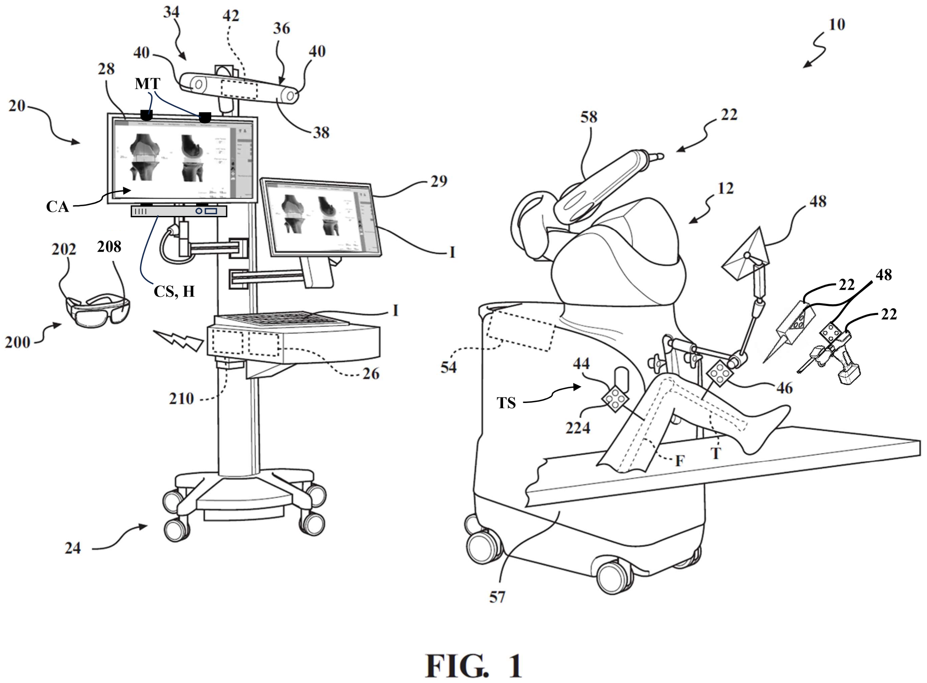

Referring to FIG. 1, a system 10 is provided. The system may be a surgical system 10 adapted for treating a patient. The surgical system 10 is shown in a surgical setting such as an operating room of a medical facility. The surgical system 10 may be used to perform any intraoperative surgical procedure on a patient. Example surgical procedures include, but are not limited to: partial knee arthroplasty, total knee arthroplasty, total hip arthroplasty, shoulder arthroplasty, spinal procedures, ankle procedures, endoscopic procedures, cranial procedures, lesion removal procedures, arthroscopic procedures, arthroscopic resection procedures, soft tissue or ligament repair procedures, neurological procedures, ENT procedures, minimally invasive MIS procedures, or the like. In the example shown in FIG. 1, the patient is undergoing a knee procedure. In addition, the following implementations describe the use of the surgical system 10 in performing a procedure in which material is removed from a femur F and/or a tibia T of a patient. However, the surgical system 10 may be used to perform any suitable procedure in which material is removed from any suitable portion of a patient's anatomy, material is added to any suitable portion of the patient's anatomy (e.g., an implant, graft, etc.), and/or in which any other control of and/or visualization of a surgical tool is desired.

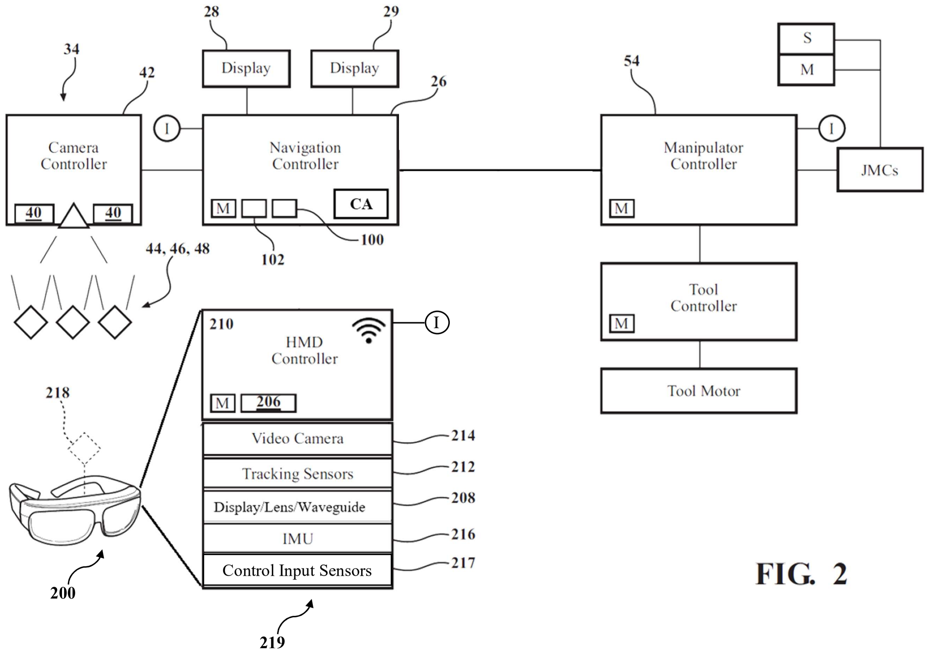

In the implementation shown, the surgical system 10 includes a manipulator 12 (e.g., surgical robot) and a navigation system 20. The navigation system 20 is set up to track movement of various objects in the operating room. Such objects include, for example, one or more surgical tools 22 and a target site TS of the patient (e.g., a femur F and a tibia T). The navigation system 20 tracks these objects for purposes of displaying their relative positions and orientations to the surgeon on a clinical application (CA) and, in some cases, for purposes of controlling or constraining movement of the surgical tool 22 relative to virtual cutting boundaries associated with the target site TS. An example control scheme for the surgical system 10 is shown in FIG. 2. The navigation system 20 may also track one or more surgical staff members. For example, the navigation system 20 may track a position of the surgical staff member during a surgical procedure, and/or a biomechanical control input (e.g., gaze/gesture) of the surgical staff member.

An end effector may be attached to the manipulator 12. The end effector may include any end effector suitable for a surgical procedure. In the instance of FIG. 1, the end effector includes a surgical tool 22 such that the surgical tool 22 is supported by the and movable by the manipulator 12. The surgical tool 22 may be any instrument for manipulating the anatomy of a patient, such as a saw, a cutting burr, a router, a reamer, an impactor, an ultrasonic aspirator, a probe, a scalpel, a trocar, a cutting tool, a drill, a dilator, a screwdriver, an intervertebral inserter, a distractor, an abrator, a discectomy tool, or the like. Additionally, or alternatively, the end effector may include an accessory and/or energy applicator, such as a saw blade, a cutting burr, a router, a reamer, an impactor, an ultrasonic aspirator, a probe, a scalpel, a trocar, a cutting tool, a drill, a dilator, a screwdriver, an intervertebral inserter, a distractor, an abrator, a discectomy tool, or the like. The accessory and energy applicator may be integrated or separately attached to the end effector. In some instances, the end effector may include a shaft, and the energy applicator may be located on an end of the shaft. The end effector may also include a cutting guide. As shown in FIG. 1, the end effector may include a tool holder, which may support any of the surgical tools 22 described above. The tool holder may be a guide tube for supporting a surgical tool 22. The surgical tool 22 may be temporarily affixed to the guide tube and/or slidable within the guide tube. The guide tube may be the guide tube further described in U.S. Provisional Patent Application No. 63/612,011, entitled, "Magnetic Spine Registration Tool", which is incorporated herein by reference. Additionally, the guide tube may the anti-skiving guide tube described in U.S. Provisional Patent Application No. 63/454,346, entitled, "Anti-Skiving Guide Tube And Surgical System Including The Same", which is incorporated herein by reference. Additionally, or alternatively, the surgical tools 22 can be actively driven or motorized by the manipulator 12. The surgical tools 22 can be hand-held and selectively coupled to the manipulator 12.

In the implementation shown, the surgical tool 22 is attached to the manipulator 12. Such an arrangement is shown in U.S. Patent No. 9,119,655, entitled, "Surgical Manipulator Capable of Controlling a Surgical Instrument in Multiple Modes," the disclosure of which is hereby incorporated by reference. In one example, the manipulator 12 has a base 57, a plurality of links 58 extending from the base 57, and a plurality of joints (not numbered) for moving the surgical tool 22 with respect to the base 57. The links 58 and joints form a robotic arm. Some or all of the joints may be passive joints or active joints. The manipulator 12 may have a serial arm or parallel arm configuration. The manipulator 12 may be floor mounted, ceiling mounted, gantry mounted, table mounted, or patient mounted. More than one manipulator 12 may be utilized.

While the surgical system 10 is illustrated in FIG. 1 as including the surgical tool 22 attached to the manipulator 12, it should be recognized that the surgical system 10 may additionally or alternatively include one or more manually operated or hand-held surgical tools 22. For example, the surgical tool 22 may include a hand-held motorized saw/drill/bur/driver, a hand-held probe/pointer/digitizer, or other suitable tool that may be held and manually operated by a surgeon. Any implementations described with reference to the use of the manipulator 12 may also apply to the use of a hand-held tool 22 with appropriate modifications.

The navigation system 20 includes one or more computer cart assemblies 24 that houses one or more navigation controllers 26. A navigation interface is in operative communication with the navigation controller 26. The navigation interface includes one or more displays 28, 29 adjustably mounted to the computer cart assembly 24 or mounted to separate carts as shown.

The clinical application CA may be displayed on one or more displays 28, 29 of the navigation system 20. The clinical application CA assists a surgeon or staff in performing the surgical procedure. The clinical application CA may have a plurality of different screens related to the surgical procedure. Such screens may include a pre-operative planning screen, an operating room setup screen, an anatomical registration screen, an intra-operative planning screen, an anatomical preparation screen, or a post-operative evaluation screen, and the like. The clinical application CA may present a navigation guidance region that displays one or more of the surgical objects tracked by a localizer 34 of the navigation system 20.

The localizer 34 communicates with the navigation controller 26. In the implementation shown, the localizer 34 is an optical localizer and includes a camera unit 36. The camera unit 36 has a housing 38 comprising an outer casing that houses one or more optical sensors 40. The optical sensors 40 may detect light signals, such as infrared (IR) signals and/or visible light signals. The camera unit 36 may be mounted to any location within the operating room to position the optical sensors 40 with a field-of-view of the below discussed trackers that, ideally, is free from obstructions. For example, the camera unit 36 may be mounted to a component of the surgical system 10, such as the computer cart assembly 24, as shown in FIG. 1. Additionally, or alternatively, the camera unit 36 may be mounted to an adjustable arm. The camera unit 36 may also be mounted to a structure of the operating room, such as a wall or a ceiling of the operating room. The camera unit 36 includes a camera controller 42 in communication with the optical sensors 40 to receive signals from the optical sensors 40. The camera controller 42 communicates with the navigation controller 26 through either a wired or wireless connection. In other implementations, the optical sensors 40 communicate directly with the navigation controller 26. Position and orientation signals and/or data are transmitted to the navigation controller 26 for purposes of tracking objects. The computer cart assembly 24, display 28, and camera unit 36 may be like those described in U.S. Patent No. 7,725,162 to Malackowski, et al. issued on May 25, 2010, entitled "Surgery System," the disclosure of which is hereby incorporated by reference. The navigation controller 26 may be a personal computer or laptop computer. Navigation controller 26 includes the displays 28, 29, central processing unit (CPU) and/or other processors, memory, and storage. The navigation controller 26 is loaded with software that converts the signals received from the camera unit 36 into data representative of the position and orientation of the objects being tracked. The navigation controller 26 includes a navigation processor. It should be understood that the navigation processor could include one or more processors to control operation of the navigation controller 26. The processors may be any type of microprocessor or multi-processor system. The term processor is not intended to limit the scope of any implementation to a single processor.

Navigation system 20 is operable with a plurality of tracking devices 44, 46, 48, also referred to herein as trackers. In the illustrated implementation, one or more trackers 44, 46 may be patient/anatomy/target site trackers, e.g., tracker 44 coupled to the femur F and another tracker 46 may be firmly affixed to the tibia T. Trackers 44, 46 are firmly affixed to sections of bone in an implementation. For example, trackers 44, 46 may be attached to the femur F and tibia T in the manner shown in U.S. Patent No. 7,725,162 to Malackowski, et al. issued on May 25, 2010, entitled "Surgery System," the disclosure of which is hereby incorporated by reference. Trackers 44, 46 may also be mounted like those shown in U.S. Patent Application No. 14/156,856, filed on January 16, 2014, entitled, "Navigation Systems and Methods for Indicating and Reducing Line-of-Sight Errors," hereby incorporated by reference herein. The trackers 44, 46 may be mounted to other tissue types or parts of the anatomy. A tool tracker 48 may be coupled to the manipulator 12 or the tool 22 at any suitable location. The tool tracker 48 may be integrated into the surgical tool 22 during manufacture or may be separately mounted to the surgical tool 22 (or to an end effector attached to the manipulator 12 of which the surgical tool 22 forms a part) in preparation for surgical procedures. The working end of the surgical tool 22, which is being tracked by virtue of the tool tracker 48, may be referred to herein as an energy applicator, and may be a rotating bur, saw, router, reamer, impactor, electrical ablation device, cut guide, tool holder, probe, or the like. Additionally, or alternatively, trackers may be coupled to human, e.g., a surgical staff member. For example, one or more trackers may be coupled to one or more body parts (e.g., a limb, a torso, a head, etc.) of the surgical staff member.

In one implementation, optical sensors 40 of the localizer 34 receive light signals from the trackers 44, 46, 48. In one example, the trackers 44, 46, 48 are passive trackers. In this implementation, each tracker 44, 46, 48 has at least three passive tracking elements or markers (e.g., reflectors) for transmitting light signals (e.g., reflecting ambient light or light emitted from the camera unit 36) to the optical sensors 40. In other implementations, active tracking markers may be employed. The active markers may be, for example, light emitting diodes transmitting light, such as infrared light. Active and passive arrangements are possible. The camera unit 36 receives optical signals from the trackers 44, 46, 48 and outputs to the navigation controller 26 signals relating to the position of the tracking markers of the trackers 44, 46, 48 relative to the localizer 34. Based on the received optical signals, navigation controller 26 generates data indicating the relative positions and orientations of the trackers 44, 46, 48 relative to the localizer 34. These relative positions may be displayed on the clinical application CA as graphical representations for surgical guidance.

In another implementation, the navigation system 20 and/or the localizer 34 are radio frequency (RF) based. For example, the navigation system 20 may comprise an RF transceiver coupled to the navigation controller 26. Here, the trackers 44, 46, 48 may comprise RF emitters or transponders, which may be passive or may be actively energized. The RF transceiver transmits an RF tracking signal, and the RF emitters respond with RF signals such that tracked states are communicated to (or interpreted by) the navigation controller 26. The RF signals may be of any suitable frequency. The RF transceiver may be positioned at any suitable location to track the objects using RF signals effectively. Furthermore, examples of RF-based navigation systems may have structural configurations that are different than the navigation system 20 illustrated throughout the drawings.

In other examples, the navigation system 20 and/or localizer 34 are electromagnetically (EM) based. For example, the navigation system 20 may comprise an EM transceiver coupled to the navigation controller 26. Here, the trackers 44, 46, 48 may comprise EM components attached thereto (e.g., various types of magnetic trackers, electromagnetic trackers, inductive trackers, and the like), which may be passive or may be actively energized. The EM transceiver generates an EM field, and the EM components respond with EM signals such that tracked states are communicated to (or interpreted by) the navigation controller 26. The navigation controller 26 may analyze the received EM signals to associate relative states thereto. Here too, examples of EM-based navigation systems may have structural configurations that are different than the navigation system 20 illustrated throughout the drawings.

In other examples, the navigation system 20 and/or the localizer 34 could be based on one or more other types of tracking systems. For example, an ultrasound-based tracking system coupled to the navigation controller 26 could be provided to facilitate acquiring ultrasound images of markers that define trackable features on the tracked objects such that tracked states are communicated to (or interpreted by) the navigation controller 26 based on the ultrasound images. As another example, a fluoroscopy-based imaging system (e.g., a C-arm) coupled to the navigation controller 26 could be provided to facilitate acquiring X-ray images of radio-opaque markers that define trackable features such that tracked states are communicated to (or interpreted by) the navigation controller 26 based on the X-ray images.

Furthermore, in some examples, a machine-vision tracking system, including a vision camera may be coupled to the navigation controller 26 and could be provided to facilitate acquiring 2D and/or 3D machine-vision images of structural features that define trackable features such that tracked states are communicated to (or interpreted by) the navigation controller 26 based on the machine-vision images. The machine vision system may be integrated into the camera unit 36, optionally in combination with infrared sensors. The machine vision system may create depth maps and may detect objects with or without trackers. The machine vision system may detect patterns, shapes, colors, computer-codes, tracking geometries, or the like. The machine vision system may detect QR codes, dynamic QR codes, point cloud imagery, and the like.

Furthermore, in some examples, a depth sensing tracking system may be coupled to the navigation controller 26 and could be provided to facilitate acquiring positional data of structural features that define trackable features such that tracked states are communicated to (or interpreted by) the navigation controller 26 based on the positional data. In some instances, the depth sensing tracking system may include a laser-based tracking system (e.g., a LiDAR system), which may include a laser scanner, a laser emitter, and a processor. In some instances, the depth sensing tracking system may include a structured light camera, including a structured light scanner, a structured light emitter, and a processor. The depth sensing tracking system may be integrated into the camera unit 36, optionally in combination with light sensors. The depth sensing tracking system may create 2D and/or 3D images of trackable features, as well depth maps and may detect objects with or without trackers.

Various types of tracking and/or imaging systems could define the localizer 34 and/or form a part of the navigation system 20 without departing from the scope of the present disclosure. Furthermore, the navigation system 20 and/or localizer 34 may have other suitable components or structure not specifically recited herein, and the various techniques, methods, and/or components described herein with respect to the optically-based navigation system 20 shown throughout the drawings may be implemented or provided for any of the other examples of the navigation system 20 described herein. For example, the navigation system 20 may utilize solely inertial tracking and/or combinations of different tracking techniques, sensors, and the like. Other configurations are contemplated.

Based on the position and orientation of the trackers 44, 46, 48 and previously loaded data, navigation controller 26 may determine the position of the working end of the surgical tool 22 (e.g., the centroid of a surgical bur) and/or the orientation of the surgical tool 22 relative to the tissue against which the working end is to be applied. In some implementations, the navigation controller 26 forwards these data to a manipulator controller 54. The manipulator controller 54 may then use the data to control the manipulator 12. This control may be like that described in U.S. Patent No. 9,119,655, entitled, "Surgical Manipulator Capable of Controlling a Surgical Instrument in Multiple Modes," or like that described in U.S. Patent No. 8,010,180, entitled, "Haptic Guidance System and Method", the disclosures of which are hereby incorporated by reference.

In one implementation, the manipulator 12 is controlled to stay within a preoperatively defined virtual boundary that may be determined by a surgical plan. The virtual boundary may be a virtual cutting boundary which defines the material of the target site TS (e.g., the femur F and tibia T) to be removed by the surgical tool 22. More specifically, each of the femur F and tibia T has a target volume of material that is to be removed by the working end of the surgical tool 22. The target volumes are defined by one or more virtual cutting boundaries. The virtual cutting boundaries define the surfaces of the bone that should remain after the procedure. The navigation system 20 tracks and controls the surgical tool 22 to ensure that the working end, e.g., the surgical bur, removes the target volume of material and does not extend beyond the virtual cutting boundary, as disclosed in U.S. Patent No. 9,119,655, entitled, "Surgical Manipulator Capable of Controlling a Surgical Instrument in Multiple Modes," the disclosure of which is hereby incorporated by reference, or as disclosed in U.S. Patent No. 8,010,180, entitled, "Haptic Guidance System and Method", the disclosure of which is hereby incorporated by reference.

The virtual cutting boundary may be defined within a virtual model of the anatomy (e.g., the femur F and tibia T), or separately from the virtual model. The virtual cutting boundary may be represented as a mesh surface, constructive solid geometry (CSG), voxels, or using other boundary representation techniques. The surgical tool 22 may be used to cut away material from the femur F and tibia T to receive an implant. The surgical implants may include unicompartmental, bicompartmental, or total knee implants as shown in U.S. Patent No. 9,381,085, entitled, "Prosthetic Implant and Method of Implantation," the disclosure of which is hereby incorporated by reference. Other implants, such as hip implants, shoulder implants, spine implants, and the like are also contemplated. The focus of the description on knee implants is provided as one example. These concepts may be equally applied to other types of surgical procedures, including those performed without placing implants.

The navigation controller 26 may also present on display 28, 29 representations indicating the relative position of the working end of the tool 22 to the tissue. These representations are provided on the clinical application CA to enable the surgical guidance during manipulation of the target site TS.

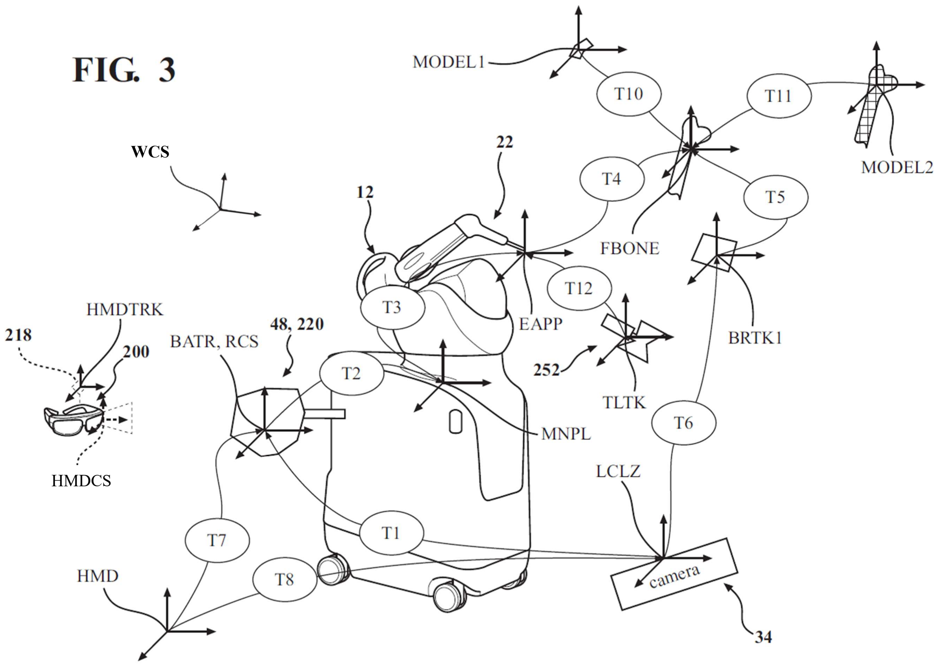

Referring to FIG. 3, tracking of objects may be conducted with reference to a localizer coordinate system LCLZ. The localizer coordinate system has an origin and an orientation (a set of x, y, and z planes). Each tracker 44, 46, 48 and object being tracked also has its own coordinate system separate from the localizer coordinate system LCLZ. Components of the navigation system 20 that have their own coordinate systems are the bone trackers 44, 46 (one of which is shown in FIG. 3) and the base tracker 48. These coordinate systems are represented as, respectively, bone tracker coordinate systems BTRK1, BTRK2 (BTRK1 shown), and base tracker coordinate system BATR. The world coordinate system WCS indicates the coordinate system of the real-world, or room, in which the objects are located.

Navigation system 20 monitors the positions of the femur F and tibia T of the patient by monitoring the position of bone trackers 44, 46 rigidly attached to bone. Femur coordinate system is FBONE and tibia coordinate system is TBONE, which are the coordinate systems of the bones to which the bone trackers 44, 46 are rigidly attached.

Prior to the start of the intraoperative procedure, preoperative images of the femur F and tibia T may be generated (or of other portions of the anatomy in other implementations). The preoperative images may be stored as two-dimensional or three-dimensional patient image data in a computer-readable storage device, such as memory within the navigation system 20. The patient image data may be based on X-ray scans or computed tomography (CT) scans of the patient's anatomy. The patient image data may then be used to generate two-dimensional images or three-dimensional models of the patient's anatomy. The pre-operative data and models may be used for surgical planning purposes and intraoperative guidance. For example, the surgical plan (e.g., tool path, resection volume, and/or virtual boundaries), may be planned relative to the virtual model. The virtual model and surgical plan may then be registered to the anatomy using any appropriate registration technique, such as pointer registration, imageless registration, or the like.

In preparation for the intraoperative procedure, the images or three-dimensional models developed from the image data are mapped to the femur coordinate system FBONE and tibia coordinate system TBONE (see transform T11). One of these models is shown in FIG. 3 with model coordinate system MODEL2. These images/models are fixed in the femur coordinate system FBONE and tibia coordinate system TBONE. As an alternative to taking preoperative images, plans for treatment may be developed in the operating room (OR) from kinematic studies, bone tracing, and other methods. The models described herein may be represented by mesh surfaces, constructive solid geometry (CSG), voxels, or using other model constructs.

During an initial phase of the intraoperative procedure, the bone trackers 44, 46 are coupled to the bones of the patient. The pose (position and orientation) of coordinate systems FBONE and TBONE are mapped to coordinate systems BTRK1 and BTRK2, respectively (see transform T5). In one implementation, a pointer instrument 252 (TLTK), such as disclosed in U.S. Patent No. 7,725,162 to Malackowski, et al., hereby incorporated by reference, having its own tracker, may be used to register the femur coordinate system FBONE and tibia coordinate system TBONE to the bone tracker coordinate systems BTRK1 and BTRK2, respectively. Given the fixed relationship between the bones and their bone trackers 44, 46, positions and orientations of the femur F and tibia T in the femur coordinate system FBONE and tibia coordinate system TBONE may be transformed to the bone tracker coordinate systems BTRK1 and BTRK2 so the localizer 34 is able to track the femur F and tibia T by tracking the bone trackers 44, 46. These pose-describing data may be stored in memory integral with both manipulator controller 54 and navigation controller 26.

The working end of the surgical tool 22 has its own coordinate system. In some implementations, the surgical tool 22 comprises a handpiece and an accessory that is removably coupled to the handpiece. The accessory may be referred to as the energy applicator and may comprise a bur, an electrosurgical tip, an ultrasonic tip, a pointer tip, or the like. Thus, the working end of the surgical tool 22 may comprise the energy applicator. The coordinate system of the surgical tool 22 is referenced herein as coordinate system EAPP. The origin of the coordinate system EAPP may represent a centroid of a surgical cutting bur, for example. In other implementations, the accessory may simply comprise a probe or other surgical tool with the origin of the coordinate system EAPP being a tip of the probe. The pose of coordinate system EAPP is registered to the pose of base tracker coordinate system BATR before the procedure begins (see transforms T1, T2, T3). Accordingly, the poses of these coordinate systems EAPP, BATR relative to each other are determined. The pose-describing data may be stored in memory integral with both manipulator controller 54 and navigation controller 26.

Referring to FIG. 2, a localization engine 100 is a software module that may be considered part of the navigation system 20. Components of the localization engine 100 run on navigation controller 26. In some implementations, the localization engine 100 may run on the manipulator controller 54. Localization engine 100 receives as inputs the signals from the localizer 34 and, in some implementations, signals from the tracker controller. Based on these signals, localization engine 100 may determine the pose of the bone tracker coordinate systems BTRK1 and BTRK2 in the localizer coordinate system LCLZ (see transform T6). Based on the same signals received for the base tracker 48, the localization engine 100 determines the pose of the base tracker coordinate system BATR in the localizer coordinate system LCLZ (see transform T1).

The localization engine 100 forwards the signals representative of the poses of trackers 44, 46, 48 to a coordinate transformer 102. Coordinate transformer 102 is a navigation system software module that runs on navigation controller 26. Coordinate transformer 102 references the data that defines the relationship between the preoperative images of the patient and the bone trackers 44, 46. Coordinate transformer 102 may also store the data indicating the pose of the working end of the surgical tool 22 relative to the base tracker 48.

During the procedure, the coordinate transformer 102 receives the data indicating the relative poses of the trackers 44, 46, 48 to the localizer 34. Based on these data, the previously loaded data, and the below-described encoder data from the manipulator 12, the coordinate transformer 102 may generate data indicating the relative positions and orientations of the coordinate system EAPP and the bone coordinate systems, FBONE and TBONE. As a result, coordinate transformer 102 generates data indicating the position and orientation of the working end of the surgical tool 22 relative to the tissue (e.g., bone) against which the working end is applied. Image signals representative of these data are forwarded to displays 28, 29 enabling the surgeon and staff to view this information. In certain implementations, other signals representative of these data may be forwarded to the manipulator controller 54 to guide the manipulator 12 and corresponding movement of the surgical tool 22.

The manipulator controller 54 may use a variety of operating modes to control the manipulator 12. For example, the manipulator controller 54 may control the manipulator 12 using semi-autonomous, automated, manual, guided-manual, and halt modes of operation. Additionally, the manipulator controller 54 may be configured to use any of the operating modes described in U.S. Patent No. 8,010,180, entitled, "Haptic Guidance System and Method", the disclosure of which is hereby incorporated by reference, to control the manipulator 12.

In the semi-autonomous and automated modes, the manipulator controller 54 directs movement of the manipulator 12 and/or surgical tool 22. For example, the manipulator controller 54 may control the manipulator 12 to facilitate movement of the surgical tool 22 in accordance with a surgical plan. In one such instance, the manipulator controller 54 may control the manipulator 12 to facilitate movement of the surgical tool 22 along a predefined tool path.

In the semi-autonomous mode, the manipulator controller 54 is capable of moving the manipulator 12 and/or surgical tool 22 free of assistance from surgical staff member. Free of assistance from surgical staff member may mean that surgical staff member does not physically move the manipulator 12 and/or surgical tool 22 by applying external forces/torques to move the manipulator 12 and/or surgical tool 22. Instead, surgical staff member may use some form of control to manage starting and stopping of movement. For example, surgical staff member may hold down a button of a control to start movement of the manipulator 12 and/or surgical tool 22 and release the button to stop movement of the manipulator 12 and/or surgical tool 22. Alternatively, surgical staff member may press a button to start movement of the manipulator 12 and/or surgical tool 22 and press a button to stop motorized movement of the manipulator 12 and/or surgical tool 22 along a predefined tool path. An example of the semi-autonomous mode is described in U.S. Patent No. 9,119,655, entitled, "Surgical Manipulator Capable of Controlling a Surgical Instrument in Multiple Modes," the disclosure of which is hereby incorporated by reference.

In the manual mode and guided-manual mode, the manipulator controller 54 is configured to control movement of the manipulator 12 and/or surgical tool 22 based on external forces/torques applied to a component of the manipulator 12 and/or surgical tool 22. The external forces/torques may also be applied to a haptic device, such as the haptic device described in U.S. Patent No. 10,350,012, entitled, "Method and Apparatus for Controlling a Haptic Device," the disclosure of which is hereby incorporated by reference.

In the manual mode, the manipulator 12 and/or surgical tool 22 may be freely moveable and surgical staff member may manually direct, and the manipulator controller 54 control, movement of the manipulator 12 and/or surgical tool 22. For instance, surgical staff member may physically contact and apply external forces/torques to a component of the manipulator 12 to direct movement of the manipulator 12 and/or surgical tool 22. Movement of the manipulator 12 and/or surgical tool 22 in the manual mode may also be constrained in relation to the virtual constraints generated by the boundary generator 66 and/or path generator 68. The manual mode is further described as the free mode in U.S. Patent No. 8,010,180, entitled, "Haptic Guidance System and Method", the disclosure of which is hereby incorporated by reference.

In the guided-manual mode, the system 10 guides movement of the surgical tool 22 along a predetermined tool path, in response to external forces/torques applied to a component of the manipulator 12 and/or the surgical tool 22. In such instances, the manipulator controller 54 controls the manipulator 12 to facilitate movement of the surgical tool 22 along the predetermined tool path in response to external forces/torques. For instance, the operator may physically contact and apply external forces/torques to a component of the manipulator 12 to guide movement of the surgical tool 22 along the predetermined tool path. The guided-manual mode relies on external forces/torques applied to the manipulator 12 and/or surgical tool 22 to advance the surgical tool 22, but such advancement, instead of merely emulating the movement that would have occurred based on the external forces/torques applied, is actively controlled to be along the predetermined tool path. As such, during guided-manual mode, a part of the surgical tool 22, such as a center point of the surgical tool 22, is constrained along the predetermined tool path. The guided-manual mode is further described in U.S. Patent No. 11,564,761, entitled, "Systems and Methods for Controlling Movement of a Surgical Tool Along a Predefined Path" the disclosure of which is hereby incorporated by reference.

In the halt mode, the manipulator controller 54 may lock the manipulator 12 to lock the surgical tool 22 in a particular pose. Additionally, in the halt mode, the manipulator controller 54 may disable the surgical tool 22, for example, by shutting off power to the surgical tool 22. The manipulator controller 54 may also disengage the surgical tool 22 from the target site TS in the halt mode. The halt mode may include characteristics of the hold mode and the safety mode as described in U.S. Patent No. 8,010,180, entitled, *"*Haptic Guidance System and Method", the disclosure of which is hereby incorporated by reference.

The manipulator controller 54 may use the position and orientation data of the surgical tool 22 and the patient's anatomy to control the manipulator 12 as described in U.S. Patent No. 9,119,655, entitled, "Surgical Manipulator Capable of Controlling a Surgical Instrument in Multiple Modes," the disclosure of which is hereby incorporated by reference, or to control the manipulator 12 as described in U.S. Patent No. 8,010,180, entitled, "Haptic Guidance System and Method", the disclosure of which is hereby incorporated by reference.

The manipulator controller 54 may have a central processing unit (CPU) and/or other manipulator processors, memory, and storage. The manipulator controller 54, also referred to as a manipulator computer, is loaded with software as described below. The manipulator processors could include one or more processors to control operation of the manipulator 12. The processors may be any type of microprocessor or multi-processor system. The term processor is not intended to limit any implementation to a single processor.

A plurality of position sensors S are associated with the plurality of links 58 of the manipulator 12. In one implementation, the position sensors S are encoders. The position sensors S may be any suitable type of encoder, such as rotary encoders. Each position sensor S is associated with a joint actuator, such as a joint motor M. Each position sensor S is a sensor that monitors the angular position of one of six motor driven links 58 of the manipulator 12 with which the position sensor S is associated. Multiple position sensors S may be associated with each joint of the manipulator 12 in some implementations. The manipulator 12 may also include a force/torque sensor coupled between the distal end of the manipulator 12 and the end effector for detecting manual forces/torques exerted on the tool 22 by an operator. The input forces/torques may be used to command movement of the manipulator 12 and/or to detect collisions with the tool 22.

In some modes, the manipulator controller 54 determines the desired location to which the surgical tool 22 should be moved. Based on this determination, and information relating to the current location (e.g., pose) of the surgical tool 22, the manipulator controller 54 determines the extent to which each of the plurality of links 58 needs to be moved in order to reposition the surgical tool 22 from the current location to the desired location. The data regarding where the plurality of links 58 are to be positioned is forwarded to joint motor controllers JMCs that control the joints of the manipulator 12 to move the plurality of links 58 and thereby move the surgical tool 22 from the current location to the desired location. In other modes, the manipulator 12 is capable of being manipulated as described in U.S. Patent No. 8,010,180, entitled, "Haptic Guidance System and Method", the disclosure of which is hereby incorporated by reference, in which case the actuators are controlled by the manipulator controller 54 to provide gravity compensation to prevent the surgical tool 22 from lowering due to gravity and/or to activate in response to a surgical staff member attempting to place the working end of the surgical tool 22 beyond a virtual boundary VB.

In order to determine the current location of the surgical tool 22, data from the position sensors S is used to determine measured joint angles. The measured joint angles of the joints are forwarded to a forward kinematics module, as known in the art. Based on the measured joint angles and preloaded data, the forward kinematics module determines the pose of the surgical tool 22 in a manipulator coordinate system MNPL (see transform T3 in FIG. 3). The preloaded data are data that define the geometry of the plurality of links 58 and joints. With this encoder-based data, the manipulator controller 54 and/or navigation controller 26 may transform coordinates from the localizer coordinate system LCLZ into the manipulator coordinate system MNPL, vice versa, or may transform coordinates from one coordinate system into any other coordinate system described herein using transformation techniques. In many cases, the coordinates of interest associated with the surgical tool 22 (e.g., the tool center point or TCP), the virtual boundaries, and the tissue being treated, are transformed into a common coordinate system for purposes of relative tracking and display.

In the implementation shown in FIG. 3, transforms T1-T6 are utilized to transform relevant coordinates into the femur coordinate system FBONE so that the position and/or orientation of the surgical tool 22 may be tracked relative to the position and orientation of the femur (e.g., the femur model) and/or the position and orientation of the volume of material to be treated by the surgical tool 22 (e.g., a cut-volume model: see transform T10). The relative positions and/or orientations of these objects may also be represented on the displays 28, 29 to enhance the surgical staff member's visualization before, during, and/or after surgery.

While the example surgical system 10 has been described with reference to the Figures, the surgical system 10 is not intended to be limited to what is specifically shown and described. For example, the surgical system 10 may not include the manipulator 12 or the navigation system 20 as specifically shown. Other systems are contemplated without departing from the scope of the disclosure.

### II. Head Mounted Device

Referring back to FIGS. 1 and 2, one or more head-mounted devices (HMDs) 200 may be incorporated into the surgical system 10. The HMD 200 may be worn by a surgical staff member and may be employed to enhance visualization before, during, and/or after surgery. The HMD 200 may be any extended reality device, which may include aspects of augmented reality, mixed reality, virtual reality, and the like. The HMD 200 may be used to visualize the same objects previously described as being visualized on the displays 28, 29, and may also be used to visualize other objects, features, instructions, warnings, etc. The HMD 200 may be used to assist with visualization of the volume of material to be cut from the patient, to help visualize the size of implants and/or to place implants for the patient, to assist with registration and calibration of objects being tracked via the navigation system 20, to see instructions and/or warnings, among other uses, as described further below.

The HMD 200 has a display 208 onto which a notification, information, and/or computer-generated content may be displayed onto a real-world view. The HMD display 208 may be positionable in front of the eyes of the surgical staff member 50 wearing the HMD, such as in front of the eyes of the surgical staff member wearing the HMD 200. In the implementation described herein, the HMD 200 provides on the HMD display 208 a computational holographic/superimposed/overlay of computer-generated content over the real-world view. In one example, the real-world view is acquired by a video camera 214 attached to the HMD. The video camera 214 produces a live video stream of the real-world and the computer-generated content may be combined into video stream of the real world. In such instances, the HMD display 208 may include one or more high-resolution displays positioned in front of the surgical staff member's eyes. The HMD display 208 may be opaque in such scenarios. The HMD 200 may include other output devices for providing notification, information, and/or computer-generated content to the surgical staff member 50. For example, the HMD 200 may include a speaker configured to generate a sound or a feedback device configured to provide haptic feedback to the surgical staff member 50.

In other implementations, the HMD 200 may implement natural see-through techniques whereby the HMD display 208 is implemented as a transparent lens/visor/waveguide provided between the surgical staff member's eyes and the real-world. The real-world view is acquired naturally by the surgical staff member's eyes, and the computer-generated content is provided on the transparent lens/visor/waveguide. Such see-through techniques may include a diffractive waveguide, holographic waveguide, polarized waveguide, reflective waveguide, or switchable waveguide.

The HMD 200 includes a support structure 202, which may be head-mountable in the form of an eyeglass or glasses, headwear or headset, or eyewear (such as a digital contact lens or lenses). The HMD 200 may include additional headbands or supports to hold the HMD 200 on the surgical staff member's head. In other implementations, the HMD 200 may be integrated into a surgical helmet or other structure worn on the surgical staff member's head, neck, and/or shoulders. Although not shown, it is contemplated that instead of the HMD 200, an extended reality display screen, such as a monitor, tablet, or hand-held display may be used, which may include similar hardware and capabilities as the HMD 200 described.

The HMD 200 may include an HMD controller 210. The HMD controller 210 may include a content generator 206 that generates the computer-generated content (also referred to as virtual images) and that transmits those images to the surgical staff member through the HMD display 208. The HMD controller 210 controls the transmission of the computer-generated content to the HMD display 208. The HMD controller 210 may be a separate computer, located remotely from the support structure 202 of the HMD 200, or may be integrated into the support structure 202 of the HMD 200. The HMD controller 210 may be a laptop computer, desktop computer, microcontroller, or the like with memory, one or more processors (e.g., multi-core processors), input devices I, output devices (fixed display in addition to HMD 200), storage capability, etc. The HMD controller 210 may be coupled to other components of the surgical system 10 through either a wired or wireless connection.

The HMD 200 comprises a plurality of tracking sensors 212 that are in communication with the HMD controller 210. In some cases, the tracking sensors 212 are provided to establish a global coordinate system for the HMD 200, also referred to as an HMD coordinate system. The HMD coordinate system is established by these tracking sensors 212, which may comprise camera sensors or other sensor types, in some cases combined with IR depth sensors (e.g., depth camera), to layout the space surrounding the HMD 200, such as using structure-from-motion techniques or the like.

The HMD controller 210 may also receive input from the tracking sensors 212 to track a position of the surgical staff member wearing the HMD 200. The tracking sensors 212 may include a photo/video camera 214 in communication with the HMD controller 210. The camera 214 may be used to obtain photographic images or video with the HMD 200, which may be useful in identifying objects or markers attached to objects, as well as producing the real-world view. For instance, the photographic images or video obtained by the HMD 200 may be used to identify body keypoints of the surgical staff member 50 such that the HMD controller 210 may determine a position of the surgical staff member 50. In some instances, the tracking sensors 212 may determine a tracked position of the surgical staff member 50 wearing the HMD 200 by determining a position and/or orientation of the HJMD 200. For example, the tracking sensors 212 may include an inertial measurement unit (IMU) 216 in communication with the HMD controller 210. The IMU 216 may comprise one or more 3-D accelerometers, 3-D gyroscopes, and the like to assist with determining a position and/or orientation of the HMD 200 (e.g., head movements/head motions) in the HMD coordinate system or to assist with tracking relative to other coordinate systems.

The HMD 200 may also comprise a control input sensors 217. In one example, the control input sensors 217 are configured to recognize biomechanical input, e.g., gesture or eye-based commands from the surgical staff member. When detecting gestures, the control input sensor 217 may sense body keypoints of the surgical staff member, such as hands, limbs, and/or fingers, to determine the surgical staff member's gesture command and controlling the HMD 200, HMD controller 210, navigation controller 26, and/or manipulator controller 54 accordingly. Gesture commands may be used for any type of input used by the system 10. The gesture commands may be detected by the HMD 200 or may be detected by the camera 214 in front of the HMD 200. The control input sensor 217 to detect gesture may include one or more cameras, infrared sensors, motion sensors, or the like. Gesture controls may include any type of hand or finger motion, including but not limited to: pinching, pointing, swiping, circling, grasping, twisting, or the like.

When detecting eye-based commands, the control input sensor 217 may sense the surgical staff member's eye position, facial expression, motion, dwell time (stare), gaze and the like, for purposes of determining the surgical staff member's intended command and controlling the HMD 200, HMD controller 210, navigation controller 26, and/or manipulator controller 54 accordingly. The eye-based commands may be detected using an eye-tracker that is positioned to face the surgical staff member's eyes, e.g., adjacent to the interior facing HMD display 208. Eye-based controls may include any type of eye-command, including but not limited to: selecting an object, moving an object, or the like. In one example, the surgical staff member 50 may select a computer-generated object displayed by the HMD 200 by staring at the object continuously for a threshold amount of time. The HMD may also control input sensors 217 in the form of a microphone for recording verbal commands. The HMD controller 210 may process the verbal commands and control the HMD display 208 in response.

Any of the described components of the HMD 200 that may sense information or process sensed information (including but not limited to, the HMD controller 210, the video camera 214, tracking sensors 212, IMU 216, and/or control input sensors 217) may be understood as being part of a "sensing system" of the HMD 220. The sensing system is identified by numeral 219 in FIG. 2.

The HMD 200 may be registered to one or more objects used in the operating room, such as the tissue being treated, the surgical tool 22, the manipulator 12, the trackers 44, 46, 48, the localizer 34, and/or the like. In one implementation, as shown in FIG. 3, a local coordinate system HMDCS is associated with the HMD 200 to move with the HMD 200 so that the HMD 200 is fixed in a known position and orientation in the HMD coordinate system. The HMD 200 may utilize the tracking sensors 212 to map the surroundings and establish the HMD coordinate system. The HMD 200 may then utilize the camera 214 to find objects in the HMD coordinate system. In some implementations, the HMD 200 uses the camera 214 to capture video images of markers attached to the objects and then determines the location of the markers in the local coordinate system HMDCS of the HMD 200 using motion tracking techniques and then converts (transforms) those coordinates to the HMD coordinate system.

In another implementation, a separate HMD tracker 218 (see FIGS. 2 and 3), similar to the trackers 44, 46, 48, could be mounted to the HMD 200 (e.g., fixed to the support structure 202). The HMD tracker 218 may have its own HMD tracker coordinate system HMDTRK that is in a known position/orientation relative to the local coordinate system HMDCS of the HMD 200. Alternatively, the tracker coordinate system HMDTRK could be calibrated to the local coordinate system HMDCS using calibration techniques. In this implementation, the local coordinate system HMDCS becomes the HMD coordinate system and the transforms T7 and T8 would instead originate therefrom. The localizer 34 could then be used to track movement of the HMD 200 via the HMD tracker 218 and transformations could then easily be calculated to transform coordinates in the local coordinate system HMDCS to the localizer coordinate system LCLZ, the femur coordinate system FBONE, the manipulator coordinate system MNPL, or other coordinate system.

Referring back to FIG. 3, a registration device 220 may be provided with a plurality of registration markers 224 (shown in FIG. 1) to facilitate registering the HMD 200 to the localizer coordinate system LCLZ. The HMD 200 locates the registration markers 224 on the registration device 220 in the HMD coordinate system via the camera 214 thereby allowing the HMD controller 210 to create a transform T7 from the registration coordinate system RCS to the HMD coordinate system. The HMD controller 210 then needs to determine where the localizer coordinate system LCLZ is with respect to the HMD coordinate system so that the HMD controller 210 may generate images having a relationship to objects in the localizer coordinate system LCLZ or other coordinate system. The registration device 220 or any technique for registering and/or calibrating the HMD 200 to another coordinate system may be like that described in US Patent No. 10,499,997, entitled "Systems and Methods for Surgical Navigation", the entire contents of which are hereby incorporated by reference in their entirety.

During use, for example, the localizer 34 and/or the navigation controller 26 may send data on an object (e.g., the cut volume model) to the HMD 200 so that the HMD 200 knows where the object is in the HMD coordinate system and may display an appropriate content in the HMD coordinate system. Any of the transforms T1-T12 may be combined to define or register the HMD coordinate system to any object. Once registration is complete, then the HMD 200 may be used to visualize computer-generated content in desired locations with respect to any objects in the operating room. Although these transforms have been described in detail, it is understood that the HMD 200 may operate without requiring any such transforms. The HMD 200 may display content without registering to the bone, or any part of the surgical system 10.

### III. Techniques for Continuous Activation of a Surgical System

### A. Introduction

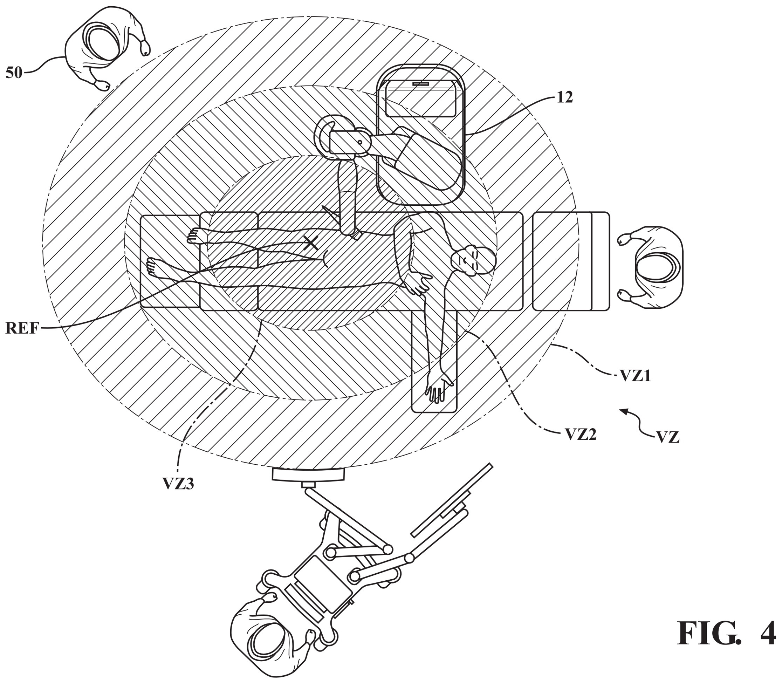

Having introduced the surgical system 10 above, this section now describes various systems, methods, software, and techniques for continuous activation of the surgical system 10. Generally, during a surgical procedure, surgical staff member provide an input to the system 10 to verify their presence. During continuous activation of the surgical system 10, surgical staff member verify their presence by being positioned within a virtual zone. Example virtual zones VZ are shown in FIG. 4. In the instance of FIG. 4, the surgical staff member 50 is positioned outside of the virtual zones VZ and the surgical system 10 has not been activated.

The surgical system 10 may include a tracking system configured to track a position of the surgical staff member 50. The tracking system may include any suitable components of the surgical system 10 for tracking the position of the surgical staff member 50. In one such example, the tracking system may include the navigation system 20. In such instances, the camera unit 36 of the localizer 34 may track a position of one or more trackers affixed to the surgical staff member 50 to track the position of the surgical staff member 50. Additionally, or alternatively, the HMD 200 may be worn by the surgical staff member 50 and the camera unit 36 may track a position of the HMD tracker 218 to track the position of the surgical staff member 50. In another example, the tracking system may include the HMD 200 and the sensing system 219 of the HMD 200 may sense a position of the HMD 200 to track the position of the surgical staff member 50. In yet another example, the tracking system may include the HMD 200 and the navigation system 20. In such instances, the sensing system 219 of the HMD 200 may sense a position of the HMD 200 and the camera unit 36 of the localizer 34 may track a position of one or more trackers to track the position of the surgical staff member 50.

The surgical system 10 may include one or more controllers (hereinafter referred to as "controllers"), which may be coupled to the manipulator 12 and to the tracking system. In this way, the controllers may be configured to control movement of the manipulator 12 based on the position of the surgical staff member 50 tracked by the tracking system. For example, in an instance where the tracking system includes the navigation system 20 and the HMD 200, the controllers may be coupled to the HMD 200 and to the navigation system 20 to receive the tracked position of the surgical staff member 50 from the HMD 200 and the navigation system 20.

The one or more controllers may include any one or more of the controllers of the surgical system 10. For example, the controllers may include one or more of the navigation controller 26, the camera controller 42, the manipulator controller 54, and/or the HMD controller 210. Accordingly, the controllers may also be located in any one or more suitable components of the surgical system 10. For example, in instances where the controllers include the HMD controller 210, the HMD 200 may house or include the controllers as the HMD 200 includes the HMD controller 210. Similarly, in instances where the controllers include the navigation controller 26 and the manipulator controller 54, the navigation system 20 (e.g. the one or more computer cart assemblies 24) and the manipulator 12 may be said to house the controllers, as the navigation system 20 includes the navigation controller 26 and the manipulator 12 includes the manipulator controller 54.

In some instances, the controllers and the tracking system may be a part of the same component. For example, the tracking system may include the navigation system 20 and the controllers may include the navigation controller 26. In such instances, the navigation system 20 may be said to include the controllers. As another example, the tracking system may include the HMD 200, and the controllers may include the HMD controller 210. In such instances, the HMD 200 may be said to include the controllers.

### B. Virtual Zone(s)

The controllers may be configured to define one or more virtual zones VZ. In the instance of FIG. 4, the controllers define a first, second, and third virtual zone VZ1, VZ2, VZ3. The surgical staff member 50 may be positioned within the virtual zones VZ during activation of the surgical system 10.

The controllers may define each virtual zone VZ by defining features of the virtual zone VZ. For example, the controllers may define each virtual zone VZ based on defining a shape, a position, and/or a size of the virtual zone VZ.

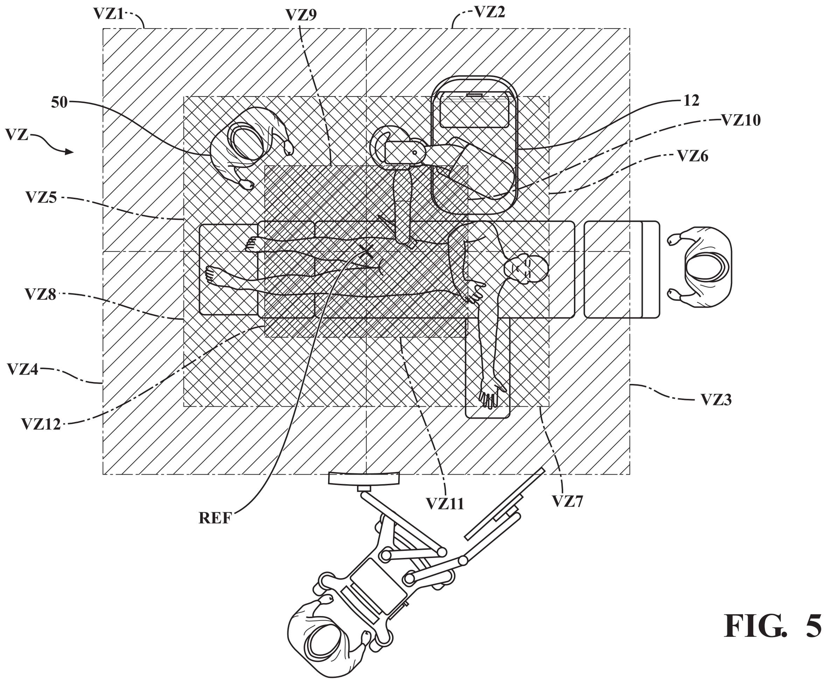

The controllers may define the virtual zones VZ to include any 2D or 3D suitable shape. In the example of FIG. 4, the controllers define the virtual zones VZ to include a circular shape. However, in other instances, the controllers may define the virtual zones VZ to include a polygonal shape. For example, in the instance of FIG. 5, the controllers define the virtual zones VZ to include a quadrilateral shape. In other instances, the controllers may define the virtual zones VZ to include any suitable geometric shape.

When the virtual zones VZ to are 2D, they may include any suitable 2D shape, such as a rectangular, square, circle, oval, triangle, any polygon, etc. Any 2D virtual zones VZ may be defined with a virtual area defined by the boundary, perimeter, or edges of the virtual zones VZ. The virtual area can be considered part of the virtual zone VZ. Alternatively, the 2D virtual zones VZ may be defined by the 2D shape boundary, irrespective of any virtual area defined within the shape boundary. Moreover, 2D virtual zones VZ may be defined such that the virtual area within the shape boundary is hollow to form an annulus or hollow shape wherein the surrounding virtual area is formed by a 2D frame. When the virtual zones VZ to are three-dimensional, they may include any suitable three-dimensional shape, such as a rectangular prism, prismatic shell, cube, cubic shell, cylinder, cylindrical shell, sphere, spherical shell, hemisphere, hemispherical shell, cone, torus, etc. Any 3D virtual zones VZ may be defined with a virtual volume defined by the boundary, virtual walls/surfaces/faces/edges of the virtual zones VZ. The virtual volume can be considered part of the virtual zone VZ. Alternatively, the 3D virtual zones VZ may be defined by the 3D shape boundary, irrespective of any virtual volume defined within the shape boundary. Moreover, 3D virtual zones VZ may be defined such that the virtual volume within the shape boundary is hollow, so as to form a 3D annuli or hollow volume wherein the surrounding virtual volume is formed by a 3D frame. Also, any 2D or 3D virtual zones VZ can be defined with a customized, complex, or irregular geometry (shape, size, perimeter, area, volume, etc.). For example, the customized geometry can be defined by any information relevant to the surgical procedure, such as, but not limited to: the actual or planned position of objects (e.g., robotic manipulator, surgical table, surgical staff member, etc.), the surgical plan, the operative limits of the robotic manipulator, the geometry/shape/dimensions of any relevant object or environmental area (e.g., robotic manipulator, surgical table, operating room), etc. Any combinations of the above are contemplated.

The controllers may define the virtual zones VZ to include any suitable position. For example, in the instance of FIGS. 4 and 5, the controllers define the virtual zones VZ such that the virtual zones VZ are defined relative to a virtual reference point REF. Specifically, in the instance of FIG. 4, the first, second, and third virtual zones VZ1, VZ2, VZ3 are centered about the virtual reference point REF; in the instance of FIG. 5, the virtual zones VZ1-VZ12 form an array, which is centered about the virtual reference point REF. The virtual reference point REF may be located relative to any component of the surgical system 10 and/or the patient. For example, the virtual reference points REF of FIGS. 4 and 5 are located relative to the manipulator 12. The virtual reference point REF may also be located relative to the target site TS of the patient (e.g., the femur F and the tibia T). In other instances, the virtual zones VZ may be located relative to any component of the surgical system 10 and/or the patient, without being defined relative to a virtual reference point REF. In such instances, the virtual zones VZ may include any suitable position relative to the component of the surgical system 10 and/or the patient. Additionally, as will be explained in greater detail below, the controllers may be configured to detect a position of the surgical staff member 50 in a virtual zone VZ. As such, the controllers may define the virtual zones VZ to include a position such that the surgical staff member 50 may be positioned within a virtual zone VZ without being positioned in an adjacent virtual zone VZ.

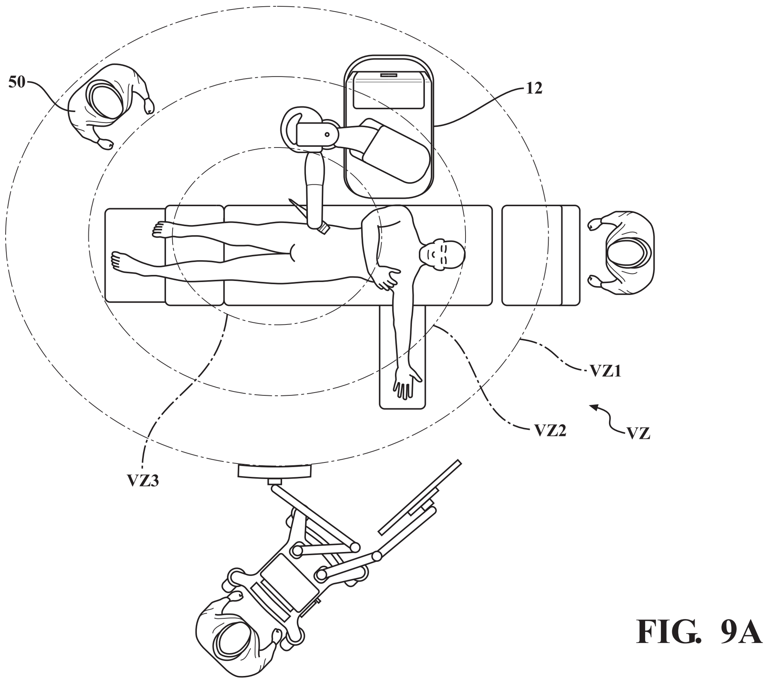

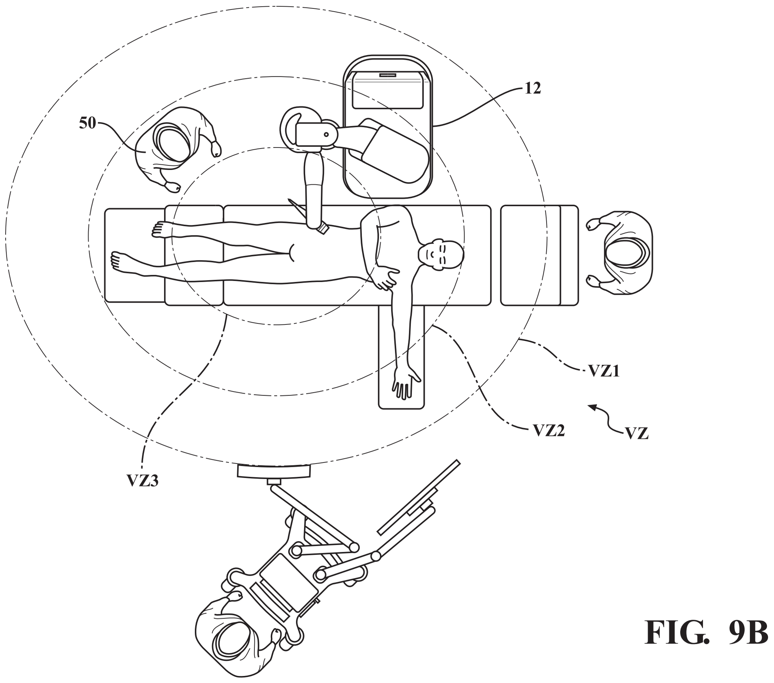

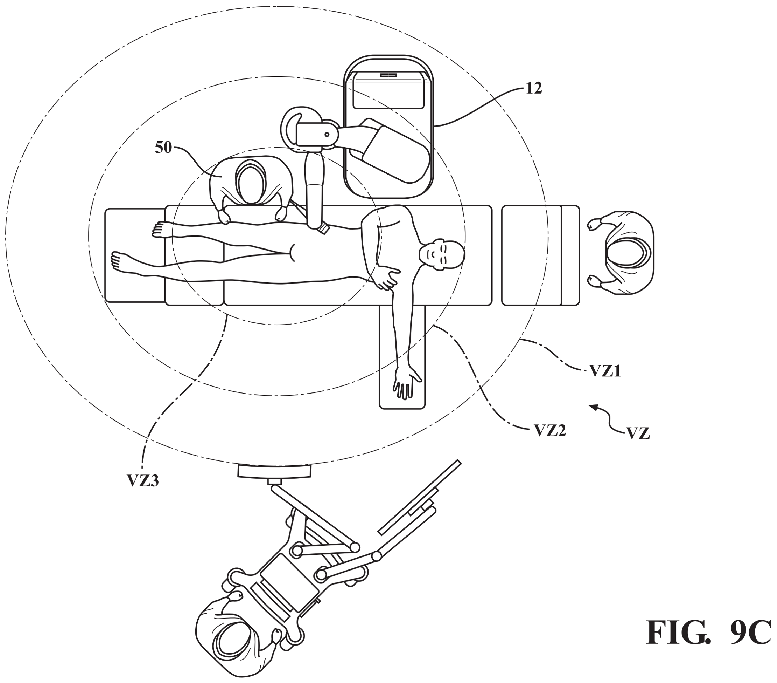

The controllers may define the virtual zones VZ to include any suitable size. As will be explained in greater detail below, the controllers may be configured to detect a position of the surgical staff member 50 in a virtual zone VZ. As such, the controllers may define the virtual zones VZ to include a suitable size such that the surgical staff member 50 may be positioned in a virtual zone VZ without being positioned in an adjacent virtual zone VZ. For example, in the example of FIG. 4, the first virtual zone VZ1 is suitably sized such that the surgical staff member 50 may be positioned in the first virtual zone VZ1 without being positioned in the second virtual zone VZ2 (see FIG. 9A). Similarly, the second virtual zone VZ2 is suitably sized such that the surgical staff member 50 may be positioned in the second virtual zone VZ2 without being positioned in either the first or third virtual zones VZ1, VZ3 (see FIG. 9B); and the third virtual zone VZ3 is suitably sized such that the surgical staff member 50 may be positioned in the third virtual zone VZ3 without being positioned in second virtual zone VZ2 (see FIG. 9C).

The controllers may also define the virtual zones VZ to include a size and position suitable for providing a secure position for the surgical staff member 50 during a surgical procedure. For example, referring to FIG. 4, the virtual zones VZ may be sized and positioned such that the surgical staff member 50 is considered to be a secure distance from the target site TS while positioned in the first virtual zone VZ1, such that the surgical staff member 50 is considered to be near the target site TS and in a "warning zone" while positioned in the second virtual zone VZ2, and such that the surgical staff member 50 is considered to be adjacent to the target site TS and in an "emergency zone" while positioned in the third virtual zone VZ3.

The controllers may also define the virtual zones VZ to include any suitable number of virtual zones. As will be explained in greater detail below, the controllers may be configured to trigger a control mode in response to detecting a position of the surgical staff member 50 in a corresponding virtual zone VZ. In some instances, each of the corresponding control modes may differ from one another. As such, the controllers may define the virtual zones VZ based on a suitable number of control modes. In the instance of FIG. 4, the controllers define three virtual zones VZ1, VZ2, VZ3. In the instance of FIG. 5, the controllers define twelve virtual zones VZ1-VZ12.

The controllers may also define the virtual zones VZ as being overlapping or non-overlapping. For example, in the instance of FIG. 4, the controllers define the virtual zones VZ1, VZ2, VZ3 to be non-overlapping. Specifically, the first virtual zone VZ1 surrounds the second and third virtual zones VZ2, VZ3 and the second virtual zone VZ3 surrounds the third virtual zone VZ3. In the instance of FIG. 5, the controllers define the virtual zones VZ1-VZ12 to overlap. Specifically, the fifth and ninth virtual zones VZ5, VZ9 are located within the first virtual zone VZ1, with the ninth virtual zone VZ9 being located within the fifth virtual zone VZ5; the sixth and tenth virtual zones VZ6, VZ10 are located within the second virtual zone VZ2, with the tenth virtual zone VZ10 being located within the sixth virtual zone VZ6; the seventh and eleventh virtual zones VZ7, VZ11 are located within the third virtual zone VZ2, with the eleventh virtual zone VZ11 being located within the seventh virtual zone VZ7; and the eighth and twelfth virtual zones VZ8, VZ12 are located within the fourth virtual zone VZ4, with the twelfth virtual zone VZ12 being located within the eighth virtual zone VZ8.

The virtual zones VZ may be defined and/or modified during any step of the surgical procedure. As will be described in greater detail below, the surgical system 10 may be operated in accordance with any one or more of the methods 300, 400, 500. One or more of the virtual zones VZ may be defined and/or modified prior to, after, and/or during any step of the methods 300, 400, 500. For example, the controllers may define a new virtual zone VZ prior to, after, and/or during any step of the methods 300, 400, 500. Additionally, the controllers may modify a feature of a defined virtual zone VZ, such as a shape, size, or position of the defined virtual zone VZ, prior to, after, and/or during any step of the methods 300, 400, 500.

### C. Triggering Control Mode(s)

### i. Introduction

The controllers may trigger a control mode for the manipulator 12 and/or surgical tool 22. A control mode may be defined by the controllers and may include control of the manipulator 12 and/or surgical tool 22 based on one or more operating parameters and/or an operating mode (e.g., semi-autonomous, automated, manual, guided-manual, and halt modes of operation).

The controllers may define a control mode based on one or more operating parameters. As such, the control mode may include control of the manipulator 12 and/or surgical tool 22 according to the one or more operating parameters. For example, the one or more operating parameters may include: a cutting speed of the surgical tool 22, a feed rate of the surgical tool 22, and a tool path of the surgical tool 22. In other instances, the one or more operating parameters may include any other suitable parameters for controlling movement of the manipulator 12 and/or surgical tool 22 during a surgical procedure. For example, the one or more operating parameters may include virtual boundaries and/or target trajectories for constraining movement of the manipulator 12 and/or surgical tool 22. As another example, the one or more operating parameters may include a position of the surgical tool 22 along a tool path and/or an acceleration of the surgical tool 22 during movement of the surgical tool 22 along a tool path. Accordingly, during a control mode, the manipulator 12 may be controlled based on any one or more of the above operating parameters.

The controllers may define a control mode based on an operating mode. As such, the control mode may include control of the manipulator 12 according to the operating mode. As previously described, the manipulator 12 may be controlled using semi-autonomous, automated, manual, guided-manual, and halt modes of operation. Accordingly, during a control mode, the manipulator 12 may be controlled in accordance with any one of the above operating modes.

The controllers may define any suitable number of control modes. In some instances, the controller may define a number of control modes based on the surgical procedure. For example, in an instance where the surgical procedure requires three different cutting speeds for the surgical tool 22, the controller may define a control mode corresponding to each cutting speed. As another example, the controllers may define a number of control modes based on a number of detectable positions and/or movements of the surgical staff member 50. For instance, as will be explained in greater detail below, the controllers may be configured to trigger a control mode in response to detecting a positioning of the surgical staff member 50 in a virtual zone VZ, in response to detecting a transitional movement of the surgical staff member 50 between virtual zones VZ, and/or in response to detecting a positioning of a limb of the surgical staff member 50 in a virtual zone VZ while the surgical staff member 50 is substantially positioned in a different virtual zone. In such instances, the controllers may define a number of control modes corresponding to the various detectable positions and/or movements of the surgical staff member 50.

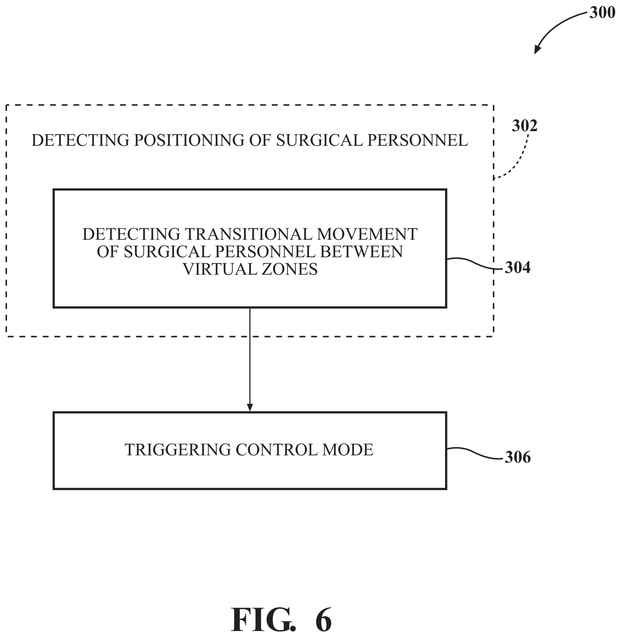

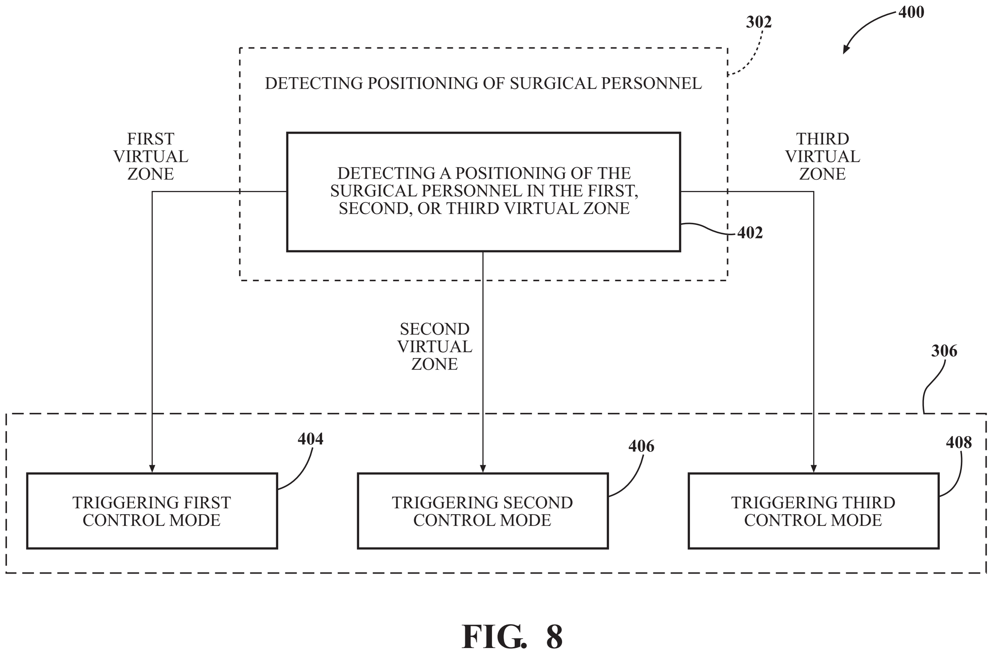

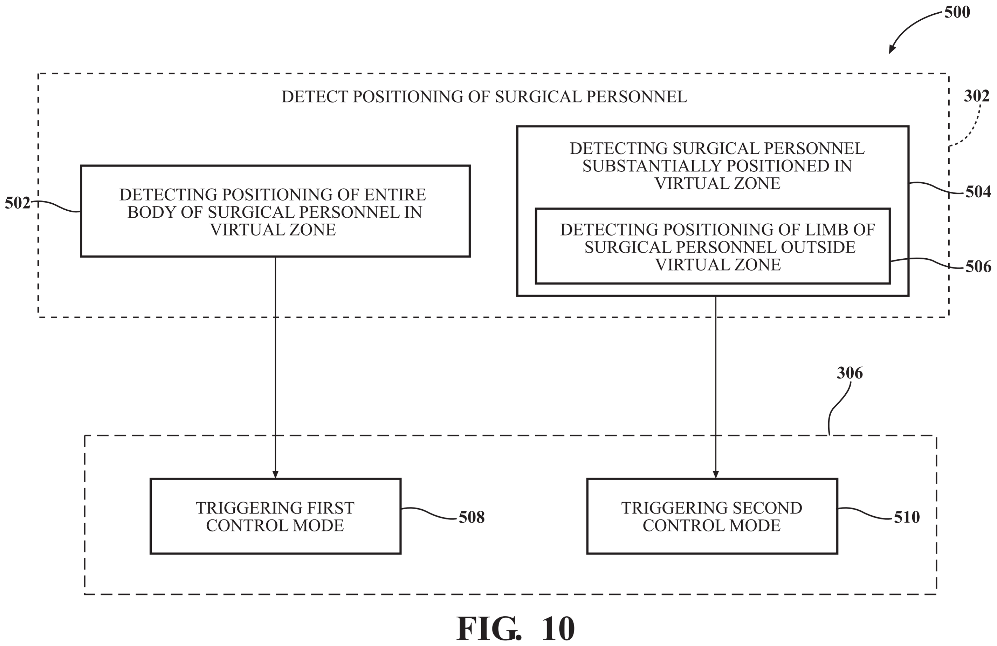

FIGS. 6, 8, and 10 illustrate a first, second, and third method 300, 400, 500 of triggering a control mode for the manipulator 12 and/or the surgical tool 22. The controllers may be configured to execute each of the first, second, and third methods 300, 400, 500. As shown, the controllers trigger a control mode during step 306 of the method 300 (FIG. 6), a control mode during steps 404, 406, 408 of the method 400 (FIG. 8), and a control mode during steps 508, 510 of the method 500 (FIG. 10).

Generally, referring to FIGS. 6, 8, and 10, for each of the methods 300, 400, 500, the controllers are configured to detect a positioning of the surgical staff member 50 during step 302 and trigger a control mode during step 306. During step 302, the controllers detect a positioning of the surgical staff member 50 based on the position of the surgical staff member 50 tracked by the tracking system. As previously stated, the tracking system is configured to track a position of the surgical staff member 50 and may include any suitable components of the surgical system 10 for tracking the position. For example, the tracking system may include the HMD 200 and/or the navigation system 20 to track a position of the surgical staff member 50. In such instances, the controllers may receive the tracked position of the surgical staff member 50 from the HMD 200 and/or the navigation system 20 and detect the position of the surgical staff member 50 based on the tracked position. During step 306, the controllers are configured to trigger a control mode in accordance with the above description of control modes.

The control modes may be defined and/or modified during any step of the surgical procedure. As previously described, the controllers may define a control mode based on one or more operating parameters and/or based on an operating mode. The controllers may modify a control mode by changing the one or more operating parameters used to control the manipulator 12 and/or surgical tool 22 during the control mode. For example, the controllers may modify a control mode such that the control mode is defined based on a cutting speed of the surgical tool 22, instead of a feed rate of the surgical tool 22. The controllers may also modify a control mode by altering control of an operating parameter of the control mode. For example, the controllers may modify a control mode by reducing a cutting speed of the surgical tool 22. The controllers may also modify a control mode by changing the operating mode of the control mode. One or more of the control modes may be defined and/or modified prior to, after, and/or during any step of the methods 300, 400, 500. The controllers may define a new control mode prior to, after, and/or during any step of the methods 300, 400, 500. Additionally, the controllers may modify a defined control mode prior to, after, and/or during any step of the methods 300, 400, 500. For instance, the controllers may modify a control mode while the control mode is being triggered.

### ii. Triggering Control Mode based on Transitional Movement

During the method 300, the controller triggers a control mode in response to detecting a transitional movement of the surgical staff member 50 between virtual zones VZ. As shown in FIG. 6, the method 300 includes the step 302 of detecting a positioning of the surgical staff member 50, and the step 306 of triggering the control mode.

During the method 300, the instance of the step 302 includes a step 304 of detecting a transitional movement of the surgical staff member 50 between the virtual zones VZ. Generally, the transitional movement of the surgical staff member 50 may be defined as a movement that alters a positioning of the surgical staff member 50 relative to the virtual zones VZ. For example, in one instance, the surgical staff member 50 may begin the transitional movement while positioned in an initial virtual zone VZ and the transitional movement may cause the surgical staff member 50 to be positioned in a different virtual zone VZ than the initial virtual zone VZ.

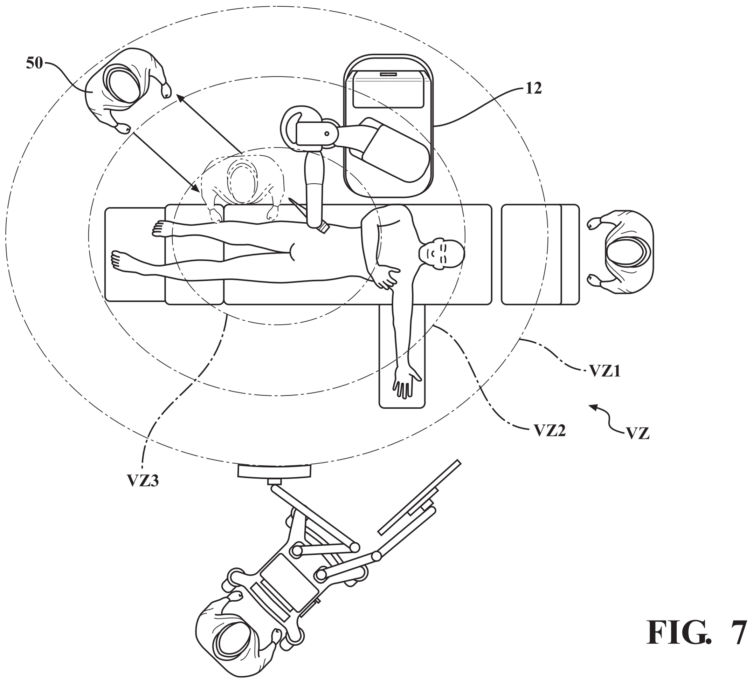

FIG. 7 provides an example of a transitional movement of the surgical staff member 50 to be detected during step 304. In the instance of FIG. 7, the transitional movement is defined as movement of the surgical staff member 50 between the first virtual zone VZ1, the second virtual zone VZ2, and the third virtual zone VZ3. Specifically, the transitional movement is defined as movement of the surgical staff member 50 from the first virtual zone VZ1 to the second virtual zone VZ2 to the third virtual zone VZ3 back to second virtual zone VZ2 and back to the first virtual zone VZ1. Referring back to FIG. 6, once the controllers detect the transitional movement of the surgical staff member 50 during step 304, the controllers may proceed to the step 306 of triggering a control mode. In the instance of FIG. 7, the controllers may trigger a control mode in response detecting movement of the surgical staff member 50 from the first virtual zone VZ1 to the second virtual zone VZ2 to the third virtual zone VZ3 back to second virtual zone VZ2 and back to the first virtual zone VZ1.

The controllers may be configured to control the manipulator 12 according to an initial control mode prior to detection of the transitional movement. For example, referring to FIG. 6, during steps 302 and 304, the controllers may be configured to control the manipulator 12 according to the initial control mode.

The initial control mode may be different from the triggered control mode.

For example, the initial and triggered control modes may include control of the manipulator 12 and/or surgical tool 22 according to one or more operating parameters. In some instances, an operating parameter of the initial control mode may be different than an operating parameter of the triggered control mode. For instance, the initial control mode may include control of the manipulator 12 and/or surgical tool 22 according to the cutting speed of the surgical tool 22 and the triggered control mode may include control of the manipulator 12 and/or surgical tool 22 according to the feed rate of the surgical tool 22. In some instances, an operating parameter of the initial and triggered control modes may be the same, however, the operating parameter may be controlled differently in the initial and triggered control modes. For instance, the initial and triggered modes may include control of the manipulator 12 and/or surgical tool 22 according to the cutting speed of the surgical tool 22. However, in the initial control mode, the manipulator 12 and/or surgical tool 22 may be controlled such that the cutting speed of the surgical tool 22 is controlled to be a first predetermined cutting speed value (e.g. 0 RPM) and in the triggered control mode the manipulator 12 and/or surgical tool 22 may be controlled such that the cutting speed of the surgical tool 22 is controlled to be a second predetermined cutting speed value (e.g. 750 RPM); where the first and second predetermined cutting speed values are different from one another.

As another example, the initial and triggered control modes may include control of the manipulator 12 and/or surgical tool 22 according to different operating modes. For example, the triggered control mode may include control of the manipulator 12 and/or surgical tool 22 according to an operating mode, such as the semi-autonomous mode, and the initial control mode may include control of the manipulator 12 and/or surgical tool 22 according to a different operating mode, such as the automated mode.

In a more specific instance, the controllers may execute the method 300 to activate the surgical system 10. Specifically, the controllers may trigger a control mode during step 306 to activate the surgical system 10. In one such instance, prior to detection of the transitional movement, the controllers may be configured to control the manipulator 12 and/or surgical tool 22 according to the halt mode. For example, referring to FIG. 7, the controllers may be configured to control the manipulator 12 and/or surgical tool 22 according to the halt mode prior to detecting that the surgical staff member 50 has completed movement from the first virtual zone VZ1 to the second virtual zone VZ2 to the third virtual zone VZ3 back to second virtual zone VZ2 and back to the first virtual zone VZ1. In such instances, once the controllers have detected the transitional movement, the controllers may be configured to trigger the control mode, which may include controlling the manipulator 12 and/or surgical tool 22 according to the automated mode. In this way, once the surgical staff member 50 performs the transitional movement and the controller detects the transitional movement, the controller may transition from the halt mode to the automated mode, activating the surgical system 10.

In various instances, the transitional movement to be detected during step 304 may vary.

As previously stated, the transitional movement may be any movement that alters a positioning of the surgical staff member 50 relative to the virtual zones VZ. In some instances, during step 304, the controllers may detect movement of the surgical staff member 50 from the first virtual zone VZ1 to the second virtual zone VZ2 to the third virtual zone VZ3 as the transitional movement. In some instances, during step 304, the controllers may detect movement of the surgical staff member 50 from the third virtual zone VZ3 to the second virtual zone VZ2 to the first virtual zone VZ1 as the transitional movement. In some instances, during step 304, the controllers may detect movement of the surgical staff member 50 from the first virtual zone VZ1 to the second virtual zone VZ2 as the transitional movement. In some instances, during step 304, the controllers may detect movement of the surgical staff member 50 from outside of the virtual zones VZ to the first virtual zone VZ1 as the transitional movement. In some instances, during step 304, the controllers may detect movement of the surgical staff member 50 from the first virtual zone VZ1 to outside of the virtual zones VZ as the transitional movement.

As another example, during step 304, the transitional movement to be detected during step 304 may be based on the number of virtual zones VZ. For example, in an instance where the controllers define two virtual zones VZ, the controllers may detect, during step 304, movement of the surgical staff member 50 from the first virtual zone to the second virtual zone and back to the first virtual zone as the transitional movement.

As yet another example, the transitional movement to be detected during step 304 may be based on an elapsing of a predetermined amount of time. For example, referring to FIG. 7, the controllers may detect, during step 304, movement of the surgical staff member 50 from the first virtual zone VZ1 to the second virtual zone VZ2 to the third virtual zone VZ3 back to second virtual zone VZ2 and back to the first virtual zone VZ1 as the transitional movement if the movement occurs within a predetermined amount of time.

### iii. Triggering Control Mode based on Positioning in Virtual Zone

During method 400, the controller triggers a control mode in response to detecting the positioning of the surgical staff member 50 in a virtual zone VZ. As shown in FIG. 8, the method 300 includes the step 302 of detecting a positioning of the surgical staff member 50, and the step 306 of triggering the control mode.

During the method 400, the instance of the step 302 includes a step 402 of detecting a positioning of the surgical staff member 50 in the first, second, or third virtual zone VZ1, VZ2, VZ3. For example, referring to FIG. 9A, the surgical staff member 50 is positioned in the first virtual zone VZ1 and, during step 402, the controller detects the positioning of the surgical staff member 50 in the first virtual zone VZ1. Referring to FIG. 9B, the surgical staff member 50 is positioned in the second virtual zone VZ2 and, during step 402, the controller detects the positioning of the surgical staff member 50 in the second virtual zone VZ2. Referring to FIG. 9C, the surgical staff member 50 is positioned in the third virtual zone VZ3 and, during step 402, the controller detects the positioning of the surgical staff member 50 in the third virtual zone VZ3.

In various instances, a definition of a positioning of the surgical staff member 50 in a virtual zone VZ may vary. For example, in the instance of FIGS. 9A-9C, the surgical staff member 50 is defined as being positioned in the virtual zones VZ1, VZ2, VZ3 if a threshold percentage of the body of the surgical staff member 50 is located in the virtual zone VZ. For example, the surgical staff member 50 may be defined as being positioned in a virtual zone VZ if greater than 75% of the body of the surgical staff member 50 is located in the virtual zone VZ. In other instances, the surgical staff member 50 may be defined as being positioned in a virtual zone VZ if a suitable number of body parts of the surgical staff member 50 are located in the virtual zone VZ. For example, the surgical staff member 50 may be defined as being positioned in a virtual zone VZ if a threshold percentage of more than three body parts (e.g., an arm, a torso, and a hip) of the surgical staff member 50 are located in the virtual zone VZ.

Referring back to FIG. 8, during the method 400, the instance of step 306 includes a step 404 of triggering a first control mode, a step 406 of triggering a second control mode, and a step 408 of triggering a third control mode. As shown, the controller triggers the first control mode in response to detecting a positioning of the surgical staff member 50 in the first virtual zone VZ1, the controller triggers the second control mode in response to detecting a positioning of the surgical staff member 50 in the second virtual zone VZ2, and the controller triggers the third control mode in response to detecting a positioning of the surgical staff member 50 in the third virtual zone VZ3.

The first, second, and third control modes may be different from one another.

For example, the first, second, and third control modes may include control of the manipulator 12 and/or surgical tool 22 according to one or more operating parameters. In some instances, an operating parameter of the first control mode may be different than an operating parameter of the second and third control modes. Similarly, an operating parameter of the second control mode may be different than an operating parameter of the first and third control modes and an operating parameter of the third control mode may be different than an operating parameter of the first and second control modes. For instance, the first control mode may include control of the manipulator 12 and/or surgical tool 22 according to the cutting speed of the surgical tool 22, the second control mode may include control of the manipulator 12 and/or surgical tool 22 according to the feed rate of the surgical tool 22, and the third control mode may include control of the manipulator 12 and/or surgical tool 22 according to the tool path of the surgical tool 22. In some instances, an operating parameter of the first, second, and third control modes may be the same, however, the operating parameter may be controlled differently in the first, second, and third control modes. For example, the first, second, and third control modes may include control of the manipulator 12 and/or surgical tool 22 according to the cutting speed of the surgical tool 22. However, in the first control mode, the manipulator 12 and/or surgical tool 22 may be controlled such that the cutting speed of the surgical tool 22 is controlled to be a first predetermined cutting speed value (e.g. 0 RPM); in the second control mode the manipulator 12 and/or surgical tool 22 may be controlled such that the cutting speed of the surgical tool 22 is controlled to be a second predetermined cutting speed value (e.g. 500 RPM); and in the third control mode the manipulator 12 and/or surgical tool 22 may be controlled such that the cutting speed of the surgical tool 22 is controlled to be a third predetermined cutting speed value (e.g. 750 RPM), where the first, second, and third predetermined cutting speed values are different from one another.

As another example, the first, second, and third control modes may include control of the manipulator 12 and/or surgical tool 22 according to different operating modes. For example, the first control mode may include control of the manipulator 12 and/or surgical tool 22 according to a first operating mode, such as the semi-autonomous mode, and the second control mode may include control of the manipulator 12 and/or surgical tool 22 according to a second different operating mode, such as the automated mode, and the third control mode may include control of the manipulator 12 and/or surgical tool 22 according to a third operating mode, such as the manual mode.

Step 402 may be defined differently based on the surgical system. For example, the step 402 may be defined based on the number of virtual zones VZ. For example, in an instance where the controllers define two virtual zones VZ, the controllers may be configured to detect a positioning of the surgical staff member 50 in the first or second virtual zone during step 402. In such instances, the step 306 may include a step of triggering a first control mode in response to the controllers detecting a positioning of the surgical staff member 40 in the first virtual zone during step 402 and a step of triggering a second control mode in response to the controllers detecting a positioning of the surgical staff member 40 in the second virtual zone during step 402. In such instances, the first control mode and the second control mode may be different from one another. As another example, in an instance where the controllers define a single virtual zone VZ, the controllers may be configured to detect a positioning of the surgical staff member 50 in the virtual zone VZ during step 402.

### iv. Triggering Control Mode based on Positioning of Limb of Surgical Staff Member

During method 500, the controller triggers a control mode in response to detecting a positioning of a limb of the surgical staff member 50. As shown in FIG. 10, the method 300 includes the step 302 of detecting a positioning of the surgical staff member 50, and the step 306 of triggering the control mode.

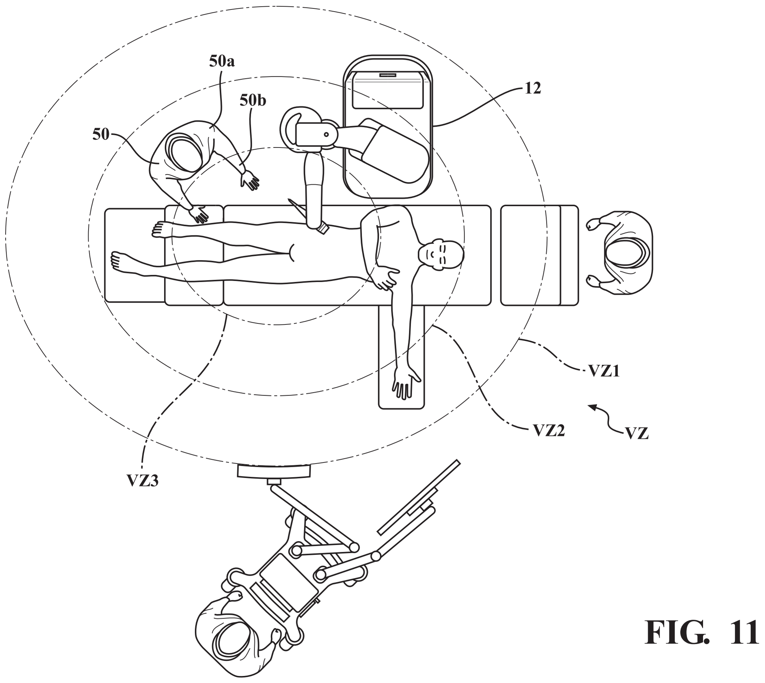

During the method 500, the instance of step 302 includes a step 504 of detecting that the surgical staff member 504 is substantially positioned in a virtual zone VZ and a step 506 of detecting a positioning of a limb (e.g. an arm or a leg) of the surgical staff member 50 outside of the virtual zone VZ while the surgical staff member 50 is substantially positioned in the virtual zone VZ. The virtual zones VZ of the method 500 may be any virtual zones VZ. For example, referring to FIG. 11, a torso 50a of the surgical staff member 50 is positioned in the first virtual zone VZ1 such that the surgical staff member 50 is substantially positioned in the first virtual zone VZ1. Additionally, a limb 50b of the surgical staff member 50 is positioned outside of the first virtual zone VZ2. Specifically, the limb 50b of the surgical staff member 50 is positioned in the second virtual zone VZ2. Referring back to FIG. 10, once the controllers detect, during step 506, a positioning of the limb 50b of the surgical staff member 50 outside of the virtual zone VZ while the surgical staff member 50 is substantially positioned in the virtual zone VZ, the controllers may proceed to the step 306 of triggering a control mode.

In some instances, during step 506, the controllers may be configured to detect a positioning of more than one limb of the surgical staff member 50 outside of the virtual zone VZ. For example, the controllers may be configured to detect, during step 506, a positioning of two limbs of the surgical staff member 50 outside of the virtual zone VZ prior to proceeding to the step 306 of triggering a control mode. In such instances, the method 500 may include an additional step where the controllers are configured to detect a positioning of more than one limb of the surgical staff member 50 outside of the virtual zone VZ.

In some instances, during step 506, the controllers may be configured to detect a positioning of one or more specific limbs of the surgical staff member 50 outside of the virtual zone VZ. For example, the controllers may be configured to detect, during step 506, a positioning of the right arm and the left leg of the surgical staff member 50 outside of the virtual zone VZ and the controllers may proceed to the step 306 of triggering a control mode in response to such a detection. As another example, the controllers may be configured to detect, during step 506, a positioning of the right arm and the left arm of the surgical staff member 50 outside of the virtual zone VZ and the controllers may proceed to the step 306 of triggering a control mode in response to such a detection. In some instances, the method 500 may include an additional step where the controllers are configured to detect a positioning of one or more specific limbs of the surgical staff member 50 outside of the virtual zone VZ.

In various instances, a definition of a substantial positioning of the surgical staff member 50 in a virtual zone VZ may vary. In the instance of FIG. 11, the surgical staff member 50 may be defined as being substantially positioned in a virtual zone VZ if a threshold percentage of the torso 50a of the surgical staff member 50 is located in the virtual zone VZ. For example, the surgical staff member 50 may be defined to be substantially positioned in a virtual zone VZ if greater than 75% of the torso 50a of the surgical staff member 50 is located in the virtual zone VZ. In other instances, the surgical staff member 50 may be defined to be substantially positioned in a virtual zone VZ if a threshold percentage of a different body part (e.g., the shoulders) of the surgical staff member 50 is located in a virtual zone VZ. In other instances, the surgical staff member 50 may be defined as being substantially positioned in a virtual zone VZ if a suitable number of body parts of the surgical staff member 50 are located in the virtual zone VZ. For example, the surgical staff member 50 may be defined as being substantially positioned in a virtual zone VZ if a threshold percentage of more than four body parts (e.g. an arm, a torso, a hip, and a head) of the surgical staff member 50 are located in the virtual zone VZ. In other instances, the surgical staff member 50 may be defined to be substantially positioned in a virtual zone VZ if a threshold percentage of the body of the surgical staff member 50 is located in a virtual zone VZ. For example, the surgical staff member 50 may be defined as being substantially positioned in a virtual zone VZ if greater than 50% of the body of the surgical staff member 50 is located in the virtual zone VZ. In other instances, the surgical staff member 50 may be defined to be substantially positioned in a virtual zone VZ based on an input from the sensing system 219 of the HMD 200. For example, the surgical staff member 50 may be defined as being substantially positioned in a virtual zone VZ based on the IMU 216 sensing a head movement and/or head motion of the surgical staff member 50.

In various instances, a definition of a limb being positioned outside of a virtual zone VZ may vary. In the instance of FIG. 11, a limb 50b of the surgical staff member 50 may be defined as being positioned outside of a virtual zone VZ if a threshold percentage of a hand/foot of the limb 50b is located outside the virtual zone VZ. For example, a limb 50b may be defined as being positioned outside of a virtual zone VZ if greater than 50% of the hand/foot of the limb 50b is located outside of the virtual zone VZ. In some instances, a limb of the surgical staff member 50 may be defined as being positioned outside of a virtual zone VZ based on a number of parts of the limb being located outside of the virtual zone VZ. For example, a limb of the surgical staff member 50 may be defined as being positioned outside of a virtual zone VZ if more than one part of the limb (e.g., both a forearm and a hand of the limb) is located outside of the virtual zone VZ. Additionally, or alternatively, a limb of the surgical staff member 50 may be defined as being positioned outside of a virtual zone VZ if a threshold percentage of the entire limb is located outside of the virtual zone VZ. In other instances, a limb of the surgical staff member 50 may be defined as being positioned outside of a virtual zone VZ based on an input from the sensing system 219 of the HMD 200. For example, the camera 214 of the HMD 200 may detect that a limb of the surgical staff member 50 is positioned outside of a virtual zone VZ.

Referring to FIG. 10, the step 302 also includes a step 502 of detecting a positioning of an entire body of the surgical staff member 50 in a virtual zone VZ. For example, FIG. 11 also provides a phantom representation of the surgical staff member 50, where an entire body of the surgical staff member 50 is located in the first virtual zone VZ1. The controllers may be configured to define a positioning of an entire body of the surgical staff member 50 in a virtual zone VZ in a variety of ways. For example, an entire body of the surgical staff member 50 may be defined as being positioned in a virtual zone VZ if greater than 90% of the body of the surgical staff member 50 is positioned in a virtual zone VZ.

Also shown in FIG. 10, the controllers may be configured to proceed to a step 508 of triggering a first control mode in response to detecting a positioning of the entire body of the surgical staff member 50 in a virtual zone VZ, and the controllers may be configured to proceed to a step 510 of triggering a second control mode in response to detecting a positioning of a limb of the surgical staff member 50 outside of the virtual zone VZ while the surgical staff member 50 is substantially positioned in the virtual zone VZ.

The first and second control modes may be different than one another. For example, the first and second control modes may include control of the manipulator 12 and/or surgical tool 22 according to one or more operating parameters. In some instances, an operating parameter of the first control mode may be different than an operating parameter of the second control mode. For instance, the first control mode may include control of the manipulator 12 and/or surgical tool 22 according to the cutting speed of the surgical tool 22 and the second control mode may include control of the manipulator 12 and/or surgical tool 22 according to the feed rate of the surgical tool 22. In some instances, an operating parameter of the first and second control modes may be the same, however, the operating parameter may be controlled differently in the first and second control modes. For instance, the first and second modes may include control of the manipulator 12 and/or surgical tool 22 according to the feed rate of the surgical tool 22. However, in the first control mode, the manipulator 12 and/or surgical tool 22 may be controlled such that the feed rate of the surgical tool 22 is controlled to be a first predetermined feed rate value (e.g. 0 mm/s) and in the second control mode the manipulator 12 and/or surgical tool 22 may be controlled such that the feed rate of the surgical tool 22 is controlled to be a second predetermined feed rate value (e.g. 3 mm/s); where the first and second predetermined feed rate values are different from one another.

As another example, the first and second control modes may include control of the manipulator 12 and/or surgical tool 22 according to different operating modes. For example, the second control mode may include control of the manipulator 12 and/or surgical tool 22 according to an operating mode, such as the guided-manual mode, and the first control mode may include control of the manipulator 12 and/or surgical tool 22 according to a different operating mode, such as the halt mode.

### v. Example Operation of Method(s)

The controllers may be configured to execute one or more of the above-described methods 300, 400, 500 in parallel or in series. In one configuration, the controllers may execute the method 300 to activate the surgical system 10, before executing both the methods 400, 500 simultaneously.

As previously stated, in some instances, the controllers may execute the method 300 to activate the surgical system 10. In the example instance provided above, the controllers may control the manipulator 12 and/or surgical tool 22 according to the halt mode prior to detection of the transitional movement during step 304 and the controllers may control the manipulator 12 and/or surgical tool 22 according to the automated mode after detection of the transitional movement during step 304. Advantageously, such a configuration of the method 300 provides security to the surgical system 10 as the surgical system 10 is not activated if the controllers have not detected the transitional movement. In the example instance of FIG. 7, the controllers do not activate the surgical system 10, unless the controllers detect that the surgical staff member 50 has completed movement from the first virtual zone VZ1 to the second virtual zone VZ2 to the third virtual zone VZ3 back to second virtual zone VZ2 and back to the first virtual zone VZ1.

Once the surgical system 10 has been activated, the controllers may execute both methods 400, 500 simultaneously.

In some instances, the control modes of the method 400 may be defined by the controller such that, as the surgical staff member 50 moves toward the target site TS, movement of the manipulator 12 and/or surgical tool 22 is slowed. Referring to FIGS. 9A-9C, the virtual zones VZ may be defined such that the surgical staff member 50 is considered to be a secure distance from the target site TS while positioned in the first virtual zone VZ1, such that the surgical staff member 50 is considered to be near the target site TS and in a "warning zone" while positioned in the second virtual zone VZ2, and such that the surgical staff member 50 is considered to be adjacent to the target site TS and in an "emergency zone" while positioned in the third virtual zone VZ3. In such instances, as the surgical staff member 50 approaches the "emergency zone", the controllers may slow operation of the manipulator 12 and/or surgical tool 22. Advantageously, such slowing provides a more secure operating environment during a surgical procedure. Additionally, such slowing allows the surgical staff member 50 to properly examine and/or diagnose potential issues with the manipulator 12, the surgical tool 22, and/or the target site TS as the surgical staff member 50 moves toward the target site TS.

As an example, during execution of the method 400, the controllers may be configured to trigger the first control mode during step 404 in response to detecting that the surgical staff member 50 is positioned in the first virtual zone VZ1 during step 402, where the manipulator 12 and/or surgical tool 22 are controlled such that the cutting speed of the surgical tool 22 is controlled to be 500 RPM in the first control mode. In such instances, the first control mode may be considered "normal control." The controllers may be configured to trigger the second control mode during step 406 in response to detecting that the surgical staff member 50 is positioned in the second virtual zone VZ2 during step 402, where the manipulator 12 and/or surgical tool 22 are controlled such that the cutting speed of the surgical tool 22 is controlled to be 250 RPM in the second control mode. In such instances, the first control mode may be considered "slowed control." The controllers may be configured to trigger the third control mode during step 408 in response to detecting that the surgical staff member 50 is positioned in the third virtual zone VZ3 during step 402, where the manipulator 12 and/or surgical tool 22 are controlled according to the halt mode in the third control mode. In such instances, the first control mode may be considered "halted control."

Advantageously, in instances where the methods 400, 500 are simultaneously executed, the method 500 may provide additional control modes to be triggered by the controllers. As previously described, during the method 400, the controllers trigger a control mode in response to a positioning of the surgical staff member 50 in a virtual zone VZ. However, during the method 500, the controllers may also trigger a control mode in response to the surgical staff member 50 being positioned between virtual zones VZ. Specifically, during the method 500, the controllers may trigger a control mode during step 510 in response to detecting a positioning of a limb of the surgical staff member 50 being outside of a virtual zone VZ, while the surgical staff member 50 is substantially positioned in the virtual zone VZ during steps 504, 506. In this way, as the surgical staff member 50 moves toward the target site TS, the controllers may trigger a control mode while the surgical staff member 50 is positioned within a virtual zone VZ and when the surgical staff member 50 is positioned between virtual zones VZ. For example, referring to FIG. 11, the controllers may trigger a control mode in response to detecting that the surgical staff member 50 is substantially positioned in the first virtual zone VZ1 and that a limb 50b of the surgical staff member 50 is positioned in the second virtual zone VZ2 (i.e. outside of the first virtual zone VZ1). In this way, as the surgical staff member 50 moves toward the target site TS, the controllers may trigger additional control modes during the method 500.

The control modes of the method 500 may be defined based on the control modes of the method 400. Referring to FIG. 10, the controllers are configured to trigger a control mode during step 508 in response to detecting that the entire body of the surgical staff member 50 is positioned within a virtual zone VZ. The control mode triggered during step 508 may be the same control mode triggered during one of steps 404, 406, 408 of the method 400 (depending on the virtual zone VZ1, VZ2, VZ3 in which the surgical staff member 50 is positioned). Additionally, just as the control modes of the method 400 may be defined such that, as the surgical staff member 50 moves toward the target site TS, movement of the manipulator 12 and/or surgical tool 22 is slowed, the control mode triggered during step 510 may be defined with the same considerations. For example, referring to FIG. 11, the controllers may be configured to trigger a control mode during step 404/step 508 in response to detecting that the entire body of the surgical staff member 50 is positioned in the first virtual zone VZ1. During the control mode triggered in step 404/508, the manipulator 12 and/or surgical tool 22 may be controlled such that the cutting speed of the surgical tool 22 is controlled to be 500 RPM. Additionally, the controllers may be configured to trigger a control mode during step 510 in response to detecting that the limb 50b of the surgical staff member 50 is positioned in the second virtual zone VZ2, while the surgical staff member 50 is substantially positioned in the first virtual zone VZ1. During the control mode triggered in step 510, the manipulator 12 and/or surgical tool 22 may be controlled such that the cutting speed of the surgical tool 22 is controlled to be 325 RPM. In this way, the control modes of the method 500 may provide increased control resolution for the controller during the slowing of movement of the manipulator 12 and/or surgical tool 22.

### D. Condition-Based Control

The controllers may detect a condition of the surgical system 10. The detected condition may be related to one or more of a surgical plan (e.g., tool path, resection volume, and/or virtual boundaries); a step of a surgical procedure; an elapsing of a predetermined amount of time; a patient; the manipulator 12; the surgical tool 22; and the surgical staff member 50.

The controllers may be configured to define and/or modify one or more features of one or more of the above-described virtual zones VZ based on the detection of a condition. Additionally, the controllers may be configured to detect a change in a condition and, in response to detection of the change of the condition, define and/or modify one or more features of one or more virtual zones VZ. The feature of the virtual zones VZ may include, but are not limited to any one or more of the following: a geometric feature (e.g., shape, size, area, volume, edges, perimeter, topology, sub-zones, shape continuity, etc.), a spatial feature (e.g., position, location, translation, rotation, orientation, etc.), a temporal feature (e.g., detection duration, time-out duration, time-windowed activation or deactivation, time-dependent size/orientation changes), and/or an interaction feature (e.g., motion profile, entry detection, exit detection, partial entry detection, partial exit detection, proximity threshold, trigger rules associated with presence, movement, or user intent, etc.). Provided below are several non-limiting examples where the controllers define and/or modify one or more virtual zones VZ based on the detection of a condition and/or a change in a condition.

In an instance where the condition is related to a surgical plan, the controllers may define and/or modify one or more features of one or more virtual zones VZ based on the resection volume. For example, the controllers may detect that the resection volume is a knee of the patient and the controllers may define and/or modify a position of the virtual zones VZ such that the virtual zones VZ are positioned about the resection volume. For instance, the controllers may define and/or modify a position of the virtual zones VZ to be centered about the resection volume.

In an instance where the condition is related to a step of a surgical procedure, the controllers may define and/or modify the one or more features of one or more virtual zones VZ based on the current step of the surgical procedure. For example, the controllers may detect a change in the condition by detecting that the surgical procedure has proceeded from a previous step to the current step. In an instance where the current step of the surgical procedure includes resection of tissue, the controllers may define and/or modify a size of the one or more virtual zones VZ to reduce a size of the virtual zones VZ. In example instances where a control mode is triggered in response to the surgical staff member 50 being positioned in a virtual zone VZ, reducing a size of the virtual zones VZ allows the surgical staff member 50 to be positioned closer to the target site TS during the triggering of the control mode. Such a reduction in the size of the virtual zones VZ allows the surgical staff member 50 to monitor the target site TS more carefully during a surgical procedure, which may be advantageous during steps including resection of tissue.

The controllers may define and/or modify the one or more features of one or more virtual zones VZ based on an elapsing of a predetermined amount of time. For example, in an instance where the predetermined amount of time indicates that the surgical procedure has just been initiated, the controllers may define and/or modify a size of the one or more virtual zones VZ to reduce a size of the virtual zones VZ. Similarly, in an instance where the predetermined amount of time indicates that the surgical procedure is almost completed, the controllers may define and/or modify a size of the one or more virtual zones VZ to reduce a size of the virtual zones VZ. In example instances where a control mode is triggered in response to the surgical staff member 50 being positioned in a virtual zone VZ, reducing a size of the virtual zones VZ allows the surgical staff member 50 to be positioned closer to the target site TS during the triggering of the control mode. Such a reduction in the size of the virtual zones VZ allows the surgical staff member 50 to monitor the target site TS more carefully during a surgical procedure, which may be advantageous during the start and end of a surgical procedure 50.

In an instance where the condition is related to the patient, the controllers may define and/or modify the virtual zones VZ based on the virtual model of the anatomy of the patient, an identity of the patient, and/or an electronic medical record of the patient. For example, in an instance where the electronic medical record of the patient indicates that the patient has a low bone density, the controllers may define and/or modify a size of the one or more virtual zones VZ to reduce a size of the virtual zones VZ. In example instances where a control mode is triggered in response to the surgical staff member 50 being positioned in a virtual zone VZ, reducing a size of the virtual zones VZ allows the surgical staff member 50 to be positioned closer to the target site TS during the triggering of the control mode. Such a reduction in the size of the virtual zones VZ allows the surgical staff member 50 to monitor the target site TS more carefully during a surgical procedure, which may be advantageous in instances where the patient has a low bone density.

In an instance where the condition is related to the manipulator 12 and/or the surgical tool 22, the controllers may define and/or modify the virtual zones VZ based on a type of the surgical tool 22, an operating parameter of the surgical tool 22, and/or a pose of the manipulator 12. For example, the controllers may detect a change in the condition by detecting that the surgical tool 22 has been changed from a rotating burr to an impactor. In such instances, the controllers may define and/or modify a size of the one or more virtual zones VZ to reduce a size of the virtual zones VZ. In example instances where a control mode is triggered in response to the surgical staff member 50 being positioned in a virtual zone VZ, reducing a size of the virtual zones VZ allows the surgical staff member 50 to be positioned closer to the target site TS during the triggering of the control mode. Such a reduction in the size of the virtual zones VZ allows the surgical staff member 50 to monitor the target site TS more carefully during a surgical procedure, which may be advantageous in instances where the surgical tool 22 has been changed form a rotating burr to an impactor.

In an instance where the condition is related to the surgical staff member 50, the controllers may define and/or modify the virtual zones VZ based on an identity of the surgical staff member 50 and/or a preference of the surgical staff member 50. For example, in an instance where the controllers detect that the surgical staff member 50 prefers to be positioned further from the target site TS, the controllers may define and/or modify a size of the virtual zones VZ to increase a size of the virtual zones VZ. In example instances where a control mode is triggered in response to the surgical staff member 50 being positioned in a virtual zone VZ, increasing a size of the virtual zones VZ allows the surgical staff member 50 to be positioned further from the target site TS during the triggering of the control mode.

The controllers may be configured to define and/or modify one or more of the above-described control modes based on the detection of a condition. Additionally, the controllers may detect a change in a condition and, in response to detection of the change of the condition, define and/or modify one or more of the control modes. As previously described, the controllers may define a control mode based on one or more operating parameters and/or based on an operating mode. The controllers may modify a control mode by changing the one or more operating parameters used to control the manipulator 12 and/or surgical tool 22 during the control mode. The controllers may also modify a control mode by altering a control of an operating parameter of the control mode. The controllers may also modify a control mode by changing the operating mode of the control mode. Provided below are several non-limiting examples where the controllers define and/or modify one or more control modes based on the detection of a condition and/or a change in a condition.

In an instance where the condition is related to a surgical plan, the controllers may define and/or modify one or more of the control modes based on the virtual boundaries. For example, the controllers may detect a change in the condition by detecting that the surgical tool 22 is nearing a virtual boundary. In such an instance, the controllers may define a control mode based on a position of the surgical tool 22 relative to the virtual boundary. For example, the controllers may define a control mode including control of the manipulator 12 and/or surgical tool 22 according to a low cutting speed of the surgical tool 22. The controllers may also modify a control mode based on a position of the surgical tool 22 relative to the virtual boundary. For example, the controllers may modify a triggered control mode by reducing the cutting speed of the surgical tool 22. Such definition/modification of control modes is advantageous in instances where the virtual boundary defines material that should remain after the procedure.

In an instance where the condition is related to a step of a surgical procedure, the controllers may define and/or modify one or more of the control modes based on the current step of the surgical procedure. For example, the controllers may detect a change in the condition by detecting that the surgical procedure has proceeded from resection of dense tissue during a previous step of the surgical procedure to resection of less dense tissue during the current step of the surgical procedure. In such an instance, the controllers may define a control mode based on the density of the tissue to be resected during the current step of the surgical procedure. For example, the controllers may define a control mode including control of the manipulator 12 and/or surgical tool 22 according to a low cutting speed of the surgical tool 22 for resection of less dense tissue during the current step of the surgical procedure. The controllers may also modify a control mode based on the density of the tissue to be resected during the current step of the surgical procedure. For example, the controllers may modify a triggered control mode by reducing the cutting speed of the surgical tool 22 for resection of less dense tissue during the current step of the surgical procedure. Advantageously, such definition/modification of control modes allows the surgical staff member 50 to resect less dense tissue more carefully.

The controllers may define and/or modify one or more of the control modes based on an elapsing of a predetermined amount of time. For example, in an instance where the predetermined amount of time indicates that the surgical procedure has just been initiated, the controllers may define and/or modify a control mode such that the control mode includes control of the manipulator 12 and/or surgical tool 22 according to a semi-autonomous mode. In this way, the controllers may define/modify the control modes such that, at the start of the surgical procedure, the surgical staff member 50 manages the starting and stopping of movement of the manipulator 12 in accordance with the semi-autonomous mode. Similarly, in an instance where the predetermined amount of time indicates that the surgical procedure is almost completed, the controllers may define and/or modify a control mode such that the control mode includes control of the manipulator 12 and/or surgical tool 22 according to a manual mode. In this way, the controllers may define/modify the control modes such that the surgical staff member 50 may manually resect material that is leftover at the end of the surgical procedure in accordance with the manual mode.

In an instance where the condition is related to the patient, the controllers may define and/or modify one or more of the control modes based on the virtual model of the anatomy of the patient, an identity of the patient, and/or an electronic medical record of the patient. For example, in an instance where the electronic medical record of the patient indicates that the patient has a low bone density, the controllers may define and/or modify a control zone to reduce a cutting speed of the surgical tool 22. Such a reduction in the cutting speed of the surgical tool 22 allows the surgical staff member 50 to more carefully resect and monitor the target site TS during a surgical procedure, which may be advantageous in instances where the patient has a low bone density.

In an instance where the condition is related to the manipulator 12 and/or the surgical tool 22, the controllers may define and/or modify one or more of the control modes based on a type of the surgical tool 22, an operating parameter of the surgical tool 22, and/or a pose of the manipulator 12. For example, the controllers may detect a change in the condition by detecting that the surgical tool 22 has been changed from a rotating burr to an impactor. In such instances, the controllers may define and/or modify a control mode such that the control mode includes control of the manipulator 12 and/or surgical tool 22 according to a semi-autonomous mode. In this way, the controllers may define/modify the control modes such that, when the surgical tool 22 has been changed to an impactor, the surgical staff member 50 manages the starting and stopping of movement of the manipulator 12 in accordance with the semi-autonomous mode.

In an instance where the condition is related to the surgical staff member 50, the controllers define and/or modify one or more of the control modes based on an identity of the surgical staff member 50 and/or a preference of the surgical staff member 50. For example, in an instance where the controllers detect that the surgical staff member 50 prefers to more carefully monitor the surgical procedure, the controllers may define and/or modify a control mode such that the control mode includes control of the manipulator 12 and/or surgical tool 22 according to a guided-manual mode. In this way, the controllers may define/modify the control modes such that, when the surgical staff member 50 prefers to more carefully monitor the surgical procedure, the surgical staff member 50 applies external forces/torques to a component of the manipulator 12 and/or surgical tool 22 in accordance with the guided-manual mode.

### E. Input Devices

The surgical system 10 may include one or more input devices I coupled to one or more components of the surgical system 10 through either a wired or wireless connection. For example, referring to FIG. 2, input devices I may be coupled to the navigation controller 26, the HMD controller 210, and/or the manipulator controller 26. The input devices I may be configured to receive an input from a user of the surgical system 10, such as the surgical staff member 50. The input devices I may then input information to components of the surgical system 10 based on the input received from the user.

The input devices I may be any suitable device for receiving the user input. For example, as shown in FIG. 1, the input devices I may include a keyboard and mouse. In other instances, the input devices I may include a footswitch, a keyboard, a user-actuatable switch, a mobile computing device, a touch screen, a microphone for voice-activation input, an optical sensor for gesture input, a wearable device, and the like. The input devices I may include a button located on the surgical tool 22 and/or a button located on a hand-held pendant device, as shown and described in U.S. Patent No. 9,119,655, entitled, "Surgical Manipulator Capable of Controlling a Surgical Instrument in Multiple Modes," the disclosure of which is hereby incorporated by reference.

The input devices I may be coupled to the navigation controller 26 through either a wired or wireless connection. For example, as shown in FIG. 1, the input devices I may include a keyboard and a mouse coupled to the navigation controller 26. The input devices I may be used to input information into the navigation controller 26 or otherwise select/control certain aspects of the navigation controller 26. For example, the surgical staff member 50 may provide an input to the input devices I to interact with the clinical application CA. For instance, the surgical staff member 50 may provide an input to the input devices I to modify or view the pre-operative planning screen, the operating room setup screen, the anatomical registration screen, the intra-operative planning screen, the anatomical preparation screen, and/or the post-operative evaluation screen of the clinical application CA. In a more specific implementation, the surgical staff member 50 may provide an input to the input devices I to modify and/or view the virtual model and/or the surgical plan (e.g., tool path, resection volume, and/or virtual boundaries).

The input devices I may be coupled to the manipulator controller 54 through either a wired or wireless connection. For example, the input devices I may include a remote-control pendant, a keyboard, a touch screen, and/or a mouse coupled to the manipulator controller 54. The input devices I may be used to input information into the manipulator controller 54 or otherwise select/control certain aspects of the manipulator controller 54. For example, the surgical staff member 50 may provide an input to the input devices I to control the manipulator 12. For instance, the surgical staff member 50 may provide an input to the input devices I to modify an operating mode (e.g., semi-autonomous, automated, manual, guided-manual, or halt modes of operation) of the manipulator 12 and/or an operating parameter of the surgical tool 22.

The input devices I may be coupled to the HMD controller 210 through either a wired or wireless connection. For example, the input devices I may include the video camera 214, tracking sensors 212, IMU 216, and/or control input sensors 217 of the HMD 200. The input devices I may also include a user interface disposed on the support structure 202 of the HMD 200, such as a button, a directional pad, and/or a user-actuatable switch. The input devices I may be used to input information into the HMD controller 210 or otherwise select/control certain aspects of the HMD controller 210. For example, the surgical staff member 50 may provide an input to the input devices I to modify or view the computer-generated content displayed on the HMD display 208. For example, as will be discussed in greater detail below, the HMD display 208 may provide a notification, information, and/or computer-generated content to the surgical staff member 50 wearing the HMD 200. In such instances, the surgical staff member 50 may provide an input to an input device I to interact with the notification, information, and/or computer-generated content.

The input devices I may also input information into the navigation controller 26, the manipulator controller 56, and/or the HMD controller 210 to define and/or modify a control mode for the manipulator 12. For example, the surgical staff member 50 may provide an input to the input devices I to modify a control mode by changing the one or more operating parameters used to control the manipulator 12 and/or surgical tool 22 during the control mode. The surgical staff member 50 may also provide an input to the input devices I to modify a control mode by altering control of an operating parameter of the control mode. The surgical staff member 50 may also provide an input to the input devices I to modify a control mode by changing the operating mode (e.g., semi-autonomous, automated, manual, guided-manual, or halt modes of operation) of the control mode.

The input devices I may also input information into the navigation controller 26, the manipulator controller 56, and/or the HMD controller 210 to define and/or modify one or more features of the virtual zones VZ. For example, the surgical staff member 50 may provide an input to the input devices I to modify a shape, a position, and/or a size of a virtual zone VZ.

In some instances, the controllers may detect a positioning of the surgical staff member 50 during step 302 based on an input provided to the input devices I. For instance, the controllers may detect a transitional movement of the surgical staff member 50 or a positioning of the surgical staff member 50 in a virtual zone VZ based on an input provided to the input devices I. In this way, the surgical staff member 50 may provide a position-based indication to the controllers using an input. For example, the controllers may detect the positioning of the surgical staff member 50 in response to the surgical staff member 50 providing a gesture/gaze input, which may be sensed by the control input sensors 217. As another example, the controllers may detect the positioning of the surgical staff member 50 in response to the tracking sensors 212 sensing a head movement and/or head motion of the surgical staff member 50.

### F. Output Devices

The surgical system 10 may include output devices, such as the displays 28, 29 and the HMD display 208. The output devices may be coupled to the navigation controller 26, the manipulator controller 54, and/or the HMD controller 210 through either a wired or wireless connection. The output devices may receive an input from the navigation controller 26, the manipulator controller 54, and/or the HMD controller 210 and provide an output to the surgical staff member 50. For example, referring to FIGS. 1 and 2, the displays 28, 29 are coupled to the navigation controller 26 and may be configured to display the clinical application CA to the surgical staff member 50. As another example, the HMD display 208 may be configured to present on the display a virtual representation of one or more virtual zones VZ with a real-world view of the manipulator 12.

The output devices may be configured to provide a notification to the surgical staff member 50. For example, the output devices may be configured to provide a notification to the surgical staff member 50 after any step of any of the methods 300, 400, 500. For instance, the output devices may be configured to provide a notification in response to detecting a positioning of the surgical staff member 50 during step 302. More specifically, the output devices may provide a notification in response to detecting transitional movement of the surgical staff member 50 during the step 304, in response to detecting a positioning of the surgical staff member 50 in a virtual zone VZ during the step 402, in response to detecting a positioning of an entire body of the surgical staff member 50 in a virtual zone VZ during step 502, and/or in response to detecting a positioning of a limb of the surgical staff member 50 being outside of a virtual zone VZ, while the surgical staff member 50 is substantially positioned in the virtual zone VZ during steps 504, 506. Additionally, the output devices may be configured to provide a notification in response to triggering a control mode during step 306.

The output devices may provide a notification by providing haptic, audible, and/or visual feedback to the surgical staff member 50. In one such instance, the output device may be the HMD display 208, which may provide a visual notification to the surgical staff member 50 wearing the HMD 200. For example, the HMD display 208 may provide a visual notification (e.g., a message box) to the surgical staff member 50 in response to detection of the position of the surgical staff member in a virtual zone VZ during step 402.

In some instances, the surgical staff member 50 may provide an input to the input devices I to interact with the output provided by the output devices. For example, the surgical staff member 50 may interact with a visual notification provided by an output device via one of the input devices I. For example, the surgical staff member 50 may address or dismiss the notification provided by the HMD display 208 via one of the input devices I. For instance, the input may be a gesture/gaze sensed by the control input sensors 217 and/or a head motion sensed by the tracking sensors 212. As another example, the HMD display 208 may highlight a virtual representation of one or more virtual zones VZ based on an input provided by one of the input devices I. For instance, the control input sensor 217 may sense the gaze of the surgical staff member 50 and determine that the surgical staff member 50 is looking in the direction of a virtual zone VZ. The HMD display 208 may then highlight the virtual representation of the virtual zone VZ.

The output devices may be any device suitable for providing haptic, audible, and/or visual feedback to the surgical staff member 50. For example, the output devices may include a display, a projector device, a speaker, a light source, a haptic device, a wearable device, and the like. In one implementation, a speaker may be configured to provide a notification by providing audible feedback to the surgical staff member 50, where a pitch, tone, and/or amplitude of the audible feedback may vary based on the notification. In another implementation, a light source may be configured to provide a notification by providing visual feedback to the surgical staff member 50, where a color, amplitude, light effect (e.g., strobing) of the light outputted by the light source may vary based on the notification. In another implementation, a haptic device may be configured to provide a notification by providing haptic feedback to the surgical staff member 50, where an amplitude, frequency, and/or vibration pattern of the vibration outputted by the haptic device may vary based on the notification. In another implementation, a wearable device may be configured to provide a notification by providing haptic feedback to the surgical staff member 50, where an amplitude, frequency, and/or vibration pattern of the vibration outputted by the wearable device may vary based on the notification.

### G. Other Implementations

In some implementations, one or more virtual zones VZ may be activated or deactivated. During activation of a virtual zone VZ, the controller may detect a positioning of the surgical staff member 50 in the virtual zone VZ and a transitional movement of the surgical staff member 50 between the virtual zone VZ and another virtual zone VZ. As follows, the controller triggers a control mode based on detecting a positioning of the surgical staff member 50 in the virtual zone VZ and based on detecting a transitional movement between the virtual zone VZ and another virtual zone VZ. During deactivation of a virtual zone VZ, the controller does not detect a positioning of the surgical staff member 50 in the virtual zone VZ and nor does the controller detect a transitional movement of the surgical staff member 50 between the virtual zone VZ and another virtual zone VZ. As follows, the controller does not trigger a control mode based on detecting a positioning of the surgical staff member 50 in the virtual zone VZ nor does the controller trigger a control mode based on detecting a transitional movement between the virtual zone VZ and another virtual zone VZ.

In some instances, the surgical staff member 50 may provide an input to the input devices I to activate or deactivate a virtual zone VZ. For example, the surgical staff member may provide a user input via a keyboard to activate or deactivate a virtual zone VZ. As another example, the input may be a gesture/gaze sensed by the control input sensors 217 and/or a head motion sensed by the tracking sensors 212.

In some implementations, the controllers may detect whether the surgical staff member 50 is distracted while the surgical staff member 50 is positioned within a virtual zone VZ. For example, the controllers may determine whether the surgical staff member 50 is distracted based on a gesture/gaze sensed by the control input sensors 217 and/or a head motion sensed by the tracking sensors 212. For instance, the control input sensors 217 may sense that the surgical staff member 50 is looking away from the direction of the surgical tool 22 and/or the target site TS and the controllers may determine that the surgical staff member 50 is distracted by comparing the time spent looking away from the direction of the surgical tool 22 with a threshold amount of time. In some instances, the controllers may detect whether the surgical staff member 50 is distracted based on the virtual zone VZ in which the surgical staff member 50 is positioned. For example, in instances where the surgical staff member 50 is positioned in a virtual zone VZ far from the target site TS, the threshold amount of time may be a greater amount of time. In instances where the surgical staff member 50 is positioned in a virtual zone VZ near the target site TS, the threshold amount of time may be a lesser amount of time. As another example, in instances where the surgical staff member 50 is positioned in a virtual zone VZ far from the target site TS, the controllers may optionally omit detection of whether the surgical staff member 50 is distracted. In instances where the surgical staff member 50 is positioned in a virtual zone VZ near the target site TS, the controllers may detect whether the surgical staff member 50 is distracted.

Several implementations have been discussed in the foregoing description. However, the implementations discussed herein are not intended to be exhaustive or limit the invention to any particular form. The terminology which has been used is intended to be in the nature of words of description rather than of limitation. Many modifications and variations are possible in light of the above teachings and the invention may be practiced otherwise than as specifically described.

The present disclosure also comprises the following CLAUSES, which may be additionally accompanied by any of the aspects or implementations provided in the summary and/or by any features otherwise found in the disclosure or the figures of the subject application.

A1. A surgical system comprising: a surgical robot that is configured to support and move a surgical tool; a tracking system that is configured to track a position of a surgical staff member; and one or more controllers coupled to the surgical robot and the tracking system and being configured to: define a first virtual zone relative to the surgical robot; define a second virtual zone relative to the surgical robot; detect a transitional movement of the surgical staff member between the first virtual zone and the second virtual zone based on the position of the surgical staff member tracked by the tracking system; and in response to detection of the transitional movement, trigger a control mode for the surgical robot. A2. The surgical system of any preceding clause, wherein the control mode for the surgical robot comprises an automated mode. A3. The surgical system of clause A2, wherein, prior to detection of the transitional movement, the one or more controllers are configured to control the surgical robot according to a halt mode; and wherein, in response to detection of the transitional movement, the one or more controllers are configured to transition from the halt mode to the automated mode. A4. The surgical system of any preceding clause, wherein the transitional movement is based on an elapsing of a predetermined amount of time. A5. The surgical system of any preceding clause, wherein the control mode is further defined as a first control mode, and wherein, prior to detection of the transitional movement, the one or more controllers are configured to control the surgical robot according to a second control mode. A6. The surgical system of clause A5, wherein: the first control mode and the second control mode each comprise control of the surgical robot according to an operating mode, the operating mode including one of: an automated mode, a manual mode, and a halt mode; and the operating mode of the first control mode is different than the operating mode of the second control mode. A7. The surgical system of any preceding clause, wherein the one or more controllers are configured to: define a third virtual zone relative to the surgical robot, wherein the transitional movement of the surgical staff member is further defined as being movement between the first virtual zone, the second virtual zone, and the third virtual zone; detect the transitional movement of the surgical staff member between the first virtual zone, the second virtual zone, and the third virtual zone based on the position of the surgical staff member tracked by the tracking system; and in response to detection of the transitional movement, trigger the control mode for the surgical robot. A8. The surgical system of any preceding clause, wherein the transitional movement of the surgical staff member is further defined as being movement from the first virtual zone to the second virtual zone and back the first virtual zone. 8. A9. The surgical system (10) of any preceding clause, wherein each of the first virtual zone and the second virtual zone are volumetric.

B1. A surgical system comprising: a surgical robot that is configured to support and move a surgical tool; a tracking system that is configured to track a position of a surgical staff member; and one or more controllers coupled to the surgical robot and the tracking system and being configured to: define a virtual zone relative to the surgical robot; detect a positioning of an entire body of the surgical staff member in the virtual zone based on the position of the surgical staff member tracked by the tracking system; in response to detection of the positioning of the entire body of the surgical staff member in the virtual zone, trigger a first control mode for the surgical robot; while the surgical staff member is substantially positioned in the virtual zone, detect a positioning of a limb of the surgical staff member outside of the virtual zone based on the position of the surgical staff member tracked by the tracking system; and in response to detection of the positioning of the limb of the surgical staff member outside of the virtual zone, trigger a second control mode for the surgical robot that is different from the first control mode. B2. The surgical system of clause B1, wherein: the one or more controllers are configured to define the first virtual zone and the second virtual zone such that the first virtual zone and the second virtual zone are defined relative to a virtual reference point located relative to the surgical robot; and the second virtual zone virtual zone is located closer to the virtual reference point than the first virtual zone. B3. The surgical system of any preceding clause, wherein the first control mode and the second control mode each comprise control of the surgical robot and/or surgical tool according to one or more operating parameters, the one or more operating parameters including: a cutting speed of the surgical tool, a feed rate of the surgical tool, and a tool path of the surgical tool. B4. The surgical system of clause B3, wherein an operating parameter of the first control mode is the same as an operating parameter of the second control mode, and wherein the operating parameter is controlled differently in the first control mode than in the second control mode. B5. The surgical system of clause B4, wherein an operating parameter of the first control mode is different than an operating parameter of the second control mode. B6. The surgical system of any preceding clause, wherein: the first control mode and the second control mode each comprise control of the surgical robot according to an operating mode, the operating mode including one of: an automated mode, a manual mode, and a halt mode; and the operating mode of the first control mode is different than the operating mode of the second control mode. B7. The surgical system (10) of any preceding clause, wherein the virtual zone is volumetric.

C1. A surgical system, comprising: a surgical robot that is configured to support and move a surgical tool; a head mounted device (HMD) that is configured to be worn by a surgical staff member, wherein the HMD comprises an HMD tracking system that is configured to track a position of the surgical staff member; and one or more controllers coupled to the surgical robot and the HMD and being configured to: define a virtual zone relative to the surgical robot based on input from the HMD tracking system; detect a positioning of the surgical staff member in the virtual zone based on the position of the surgical staff member tracked by the HMD tracking system; and in response to detection of the positioning of the surgical staff member in the virtual zone, trigger a control mode for the surgical robot. C2. The surgical system of clause C1, wherein the HMD comprises a display that is positionable in front of the eyes of a user of the HMD, and wherein the HMD is configured to present on the display a virtual representation of the virtual zone with a real-world view of the surgical robot. C3. The surgical system of clause C2, wherein the HMD is configured to present on the display a virtual notification in response to detection of the position of the surgical staff member in the virtual zone. C4. The surgical system of any preceding clause, wherein the HMD comprises an input device that is configured to receive an input from the surgical staff member, and the one or more controllers are configured to modify the virtual zone based on the input. C5. The surgical system of any preceding clause, wherein the virtual zone is further defined as a first virtual zone, wherein the control mode is further defined as a first control mode, and wherein the one or more controllers are configured to: define a second virtual zone relative to the surgical robot based on input from the HMD tracking system; define a third virtual zone relative to the surgical robot based on input from the HMD tracking system; in response to detection of the positioning of the surgical staff member in the second virtual zone, trigger a second control mode for the surgical robot; and in response to detection of the positioning of the surgical staff member in the third virtual zone, trigger a third control mode for the surgical robot. C6. The surgical system of clause C5, wherein the HMD comprises a display that is positionable in front of the eyes of a user of the HMD, and wherein the HMD is configured to present on the display a virtual representation of at least one of the first virtual zone, the second virtual zone, and the third virtual zone with a real-world view of the surgical robot. C7. The surgical system (10) of any preceding clause, wherein the virtual zone is volumetric.

## Claims

1. A surgical system (10) comprising:
a surgical robot (12) configured to support and move a surgical tool (22);
a tracking system (20, 34, 200) configured to track a position of a surgical staff member (50); and
one or more controllers (26, 42, 54, 210) coupled to the surgical robot (12) and the tracking system (20, 34, 200) and being configured to:
define a first virtual zone (VZ1) relative to the surgical robot (12);
define a second virtual zone (VZ2) relative to the surgical robot (12);
detect a positioning of the surgical staff member (50) in the first virtual zone (VZ1) based on the position of the surgical staff member (50) tracked by the tracking system (20, 34, 200);
in response to detection of the positioning of the surgical staff member (50) in the first virtual zone (VZ1), trigger a first control mode for the surgical robot (12);
detect a positioning of the surgical staff member (50) in the second virtual zone (VZ2) based on the position of the surgical staff member (50) tracked by the tracking system (20, 34, 200); and
in response to detection of the positioning of the surgical staff member (50) in the second virtual zone (VZ2), trigger a second control mode for the surgical robot (12) that is different from the first control mode.

2. The surgical system (10) of claim 1, wherein the first control mode and the second control mode each comprise control of the surgical robot (12) and/or the surgical tool (22) according to one or more operating parameters, the one or more operating parameters including: a cutting speed of the surgical tool (22), a feed rate of the surgical tool (22), and a tool path of the surgical tool (22).

3. The surgical system (10) of claim 2, wherein an operating parameter of the first control mode is the same as an operating parameter of the second control mode, and wherein the operating parameter is controlled differently in the first control mode than in the second control mode.

4. The surgical system (10) of any one of claims 2 to 3, wherein an operating parameter of the first control mode is different than an operating parameter of the second control mode.

5. The surgical system (10) of any preceding claim, wherein:
the first control mode and the second control mode each comprise control of the surgical robot (12) according to an operating mode, the operating mode including one of: an automated mode, a manual mode, and a halt mode; and
the operating mode of the first control mode is different than the operating mode of the second control mode.

6. The surgical system (10) of any preceding claim, wherein:
the one or more controllers (26, 42, 54, 210) are configured to define the first virtual zone (VZ1) and the second virtual zone (VZ2) such that the first virtual zone (VZ1) and the second virtual zone (VZ2) are defined relative to a virtual reference point (REF) located relative to the surgical robot (12); and
the first virtual zone (VZ1) and the second virtual zone (VZ2) are nonoverlapping.

7. The surgical system (10) of any preceding claim, wherein the first virtual zone (VZ1) surrounds the second virtual zone (VZ2).

8. The surgical system (10) of any preceding claim, wherein each of the first virtual zone (VZ1) and the second virtual zone (VZ2) are volumetric.

9. The surgical system (10) of any preceding claim, wherein the one or more controllers (26, 42, 54, 210) are configured to:
define a third virtual zone (VZ3) relative to the surgical robot (12);
detect a positioning of the surgical staff member (50) in the third virtual zone (VZ3) based on the position of the surgical staff member (50) tracked by the tracking system (20, 34, 200); and
in response to detection of the positioning of the surgical staff member (50) in the third virtual zone (VZ3), trigger a third control mode for the surgical robot (12) that is different from the first control mode and the second control mode.

10. The surgical system (10) of any preceding claim, wherein the one or more controllers (26, 42, 54, 210) are configured to detect a condition related to one or more of: a surgical plan; a step of a surgical procedure; an elapsing of a predetermined amount of time; a patient; the surgical robot (12); the surgical tool (22); and the surgical staff member (50).

11. The surgical system (10) of claim 10, wherein the one or more controllers (26, 42, 54, 210) are configured to define a feature of the first virtual zone (VZ1) and/or a feature of the second virtual zone (VZ2) based on detection of the condition,
and optionally,
wherein the feature comprises one or more of: a geometric feature, a spatial feature, a temporal feature, or an interaction feature.

12. The surgical system (10) of claim 11, wherein the one or more controllers (26, 42, 54, 210) are configured to:
detect a change in the condition; and
in response to detection of the change of the condition, modify the feature of the first virtual zone (VZ1) and/or the feature of the second virtual zone (VZ2).

13. The surgical system (10) of any one of claims 10 to 12, wherein the one or more controllers (26, 42, 54, 210) are configured to:
define the first control mode and/or the second control mode based on the condition;
and optionally,
detect a change in the condition; and
in response to detection of the change of the condition, modify the first control mode and/or the second control mode.

14. The surgical system (10) of any preceding claim, wherein:
the tracking system (200) comprises a head-mounted device (HMD)(200) and the HMD (200) comprises the one or more controllers (210);
and optionally,
the HMD (200) comprises a display (208) that is positionable in front of the eyes of a user of the HMD (200), and wherein the HMD (200) is configured to present on the display (208) a virtual representation of the first virtual zone (VZ1) and the second virtual zone (VZ2) combined with a real-world view of the surgical robot (12).

15. The surgical system (10) of any one of claims 1 to 14, wherein:
the tracking system (20, 34, 200) comprises a navigation system (20) comprising a camera unit (36), and wherein the navigation system (20) comprises the one or more controllers (26, 42); or
the tracking system (20, 34, 200) comprises a head-mounted device (HMD) and a navigation system (20) comprising a camera unit (36), and wherein the one or more controllers (26, 42, 210) are coupled to the HMD and to the navigation system (20).
